# EUROPEAN PATENT SPECIFICATION

(11) **EP 1 113 015 B1**
(45) Date of publication and mention of the grant of the patent: **15.09.2004**
(21) Application number: 00311130.9
(22) Date of filing: 13.12.2000
(51) Int. Cl.: C07D 403/10, A61K 31/402, A61P 25/24

(54) **Optically active 3-((2-piperazinyl-phenyl)methyl)-1-(4-(trifluoromethyl)-phenyl)-2-pyrrolidinone compounds as 5-HT1D receptor selective antagonists**
Optisch aktive 3-((2-piperazinyl-phenyl)methyl)-1-(4-(trifluoromethyl)-phenyl)-2-pyrrolidinone als selektive 5-HT1D Rezeptor Antagonisten
Dérivés de 3-((2-pipérazinyl-phényle)méthyle)-1-(4-(trifluorométhyl)-phényl)-2-pyrrolidinone optiquement actifs utilisés en tant qu'antagonistes sélectifs du recepteurs de 5-HT 1D

(30) Priority: 29.12.1999 US 173437 P
(43) Date of publication of application: 04.07.2001
(73) Proprietor: Pfizer Products Inc., Groton, Connecticut 06340 (US)
(72) Inventor: Adam, Mavis Diane, Groton, Connecticut 06340 (US); Caron, Stephane, Groton, Connecticut 06340 (US); Howard, Harry Ralph, Jr., Groton, Connecticut 06340 (US)
(74) Representative: Duncan, Garreth Andrew

(56) References cited:
- EP-A- 0 490 772
- EP-A- 0 574 313
- WO-A-91/18602
- WO-A-97/36867
- GLENNON R A ET AL: "5-HT 1D SEROTONIN RECEPTORS: RESULTS OF A STRUCTURE-AFFINITY INVESTIGATION" DRUG DEVELOPMENT RESEARCH,US,NEW YORK, NY, vol. 22, no. 1, 1991, pages 25-36, XP000576381 ISSN: 0272-4391
- GLENNON R A: "CONCEPTS FOR THE DESIGN OF 5-HT1A SEROTONIN AGONISTS AND ANTAGONISTS" DRUG DEVELOPMENT RESEARCH,US,NEW YORK, NY, vol. 26, no. 3, 1992, pages 251-274, XP000471712 ISSN: 0272-4391

## Description

The present invention relates to optically active 3-[(2-piperazinylphenyl)methyl]-1-[4-(trifluoromethyl)phenyl]-2-pyrrolidinones and pharmaceutically acceptable salts thereof, to processes for their preparation, to isotopically-labeled analogs thereof, to pharmaceutical compositions comprising them and to their medicinal use. More particularly, the compound, (-)-3-[[2-(4-methyl-1-piperazinyl)phenyl]methyl]-1-[4-(trifluoromethyl)phenyl]-2-pyrrolidinone is a selective antagonist of the 5-HT_{1D} receptor. The compounds of the invention are useful in treating depression, obsessive-compulsive disorder (OCD) and diseases, disorders or conditions for which a 5-HT_{1D} receptor selective antagonist is therapeutically indicated.

European Patent Publication 434,561, published on June 26, 1991, refers to 7-alkyl, alkoxy, and hydroxy substituted-1-(4-substituted-1-piperazinyl)-naphthalenes. The compounds are referred to as 5-HT₁ agonists and antagonists useful for the treatment of migraine, depression, anxiety, schizophrenia, stress and pain. European Patent Publication 343,050, published on November 23,1989, refers to 7-unsubstituted, halogenated, and methoxy substituted-1-(4-substituted-1-piperazinyl)-naphthalenes as useful 5-HT_{1A} ligand therapeutics.

Glennon *et al*. in "5-HT_{1D} Serotonin Receptors", *Drug Dev. Res.,* **22**:25-36 (1991) refers to 7-methoxy-1-(11-piperazinyl)-naphthalene as a useful 5-HT₁ ligand. Another Glennon article "Serotonin Receptors: Clinical Implications", *Neuroscience and Behavioral Reviews,* **14**:35-47 (1990), refers to the pharmacological effects associated with serotonin receptors including appetite suppression, thermoregulation, cardiovascular/hypotensive effects, sleep, psychosis, anxiety, depression, nausea, emesis, Alzheimer's disease, Parkinson's disease and Huntington's disease.

Ligands with high affinity for the 5-HT₁ receptors are well recognized as having therapeutic value for the treatment of human conditions caused by serotonin imbalance. World Patent Application WO 95/31988, published November 30, 1995, refers to the use of 5-HT_{1D} antagonist in combination with a 5-HT_{1A} antagonist to treat CNS diseases, disorders or conditions such depression, generalized anxiety, panic disorder, agoraphobia, social phobias, obsessive-compulsive disorder, post-traumatic stress disorder, memory disorders, anorexia nervosa and bulimia nervosa, Parkinson's disease, tardive dyskinesias, endocrine disorders such as hyperprolactinaemia, vasospasm (particularly in the cerebral vasculature) and hypertension, disorders of the gastrointestinal tract where changes in motility and secretion are involved, as well as sexual dysfunction.

WO 97/36867, published October 7, 1997 recites benzyl(idene)lactam derivatives and their use as selective (ant)agonists of 5-HT_{1A} and/or 5-HT_{1D} receptors and specifically recites racemic 3-[[2-(4-methyl-1-piperazinyl)phenyl]methyl]-1-[4-(trifluoromethyl)phenyl]-2-pyrrolidinone. Nonetheless, the optically active compounds of the present invention exhibit a very high level of activity as selective antagonists of the 5-HT_{1D} receptors.

### SUMMARY OF THE INVENTION

The present invention relates to a compound of formula II: 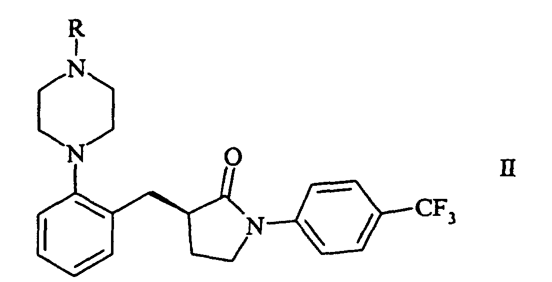 wherein R is CH₃, and pharmaceutically acceptable salts thereof. In the compound of formula II, the absolute stereochemistry at the 3-position is (*S*).

In addition, the present invention relates to mixtures of the compound of formula II, ie (-)-3(*S*)-[[2-(4-methyl-1-piperazinyl)phenyl]methyl]-1-[4-(trifluoromethyl)phenyl]-2-pyrrolidinone with its (+)-(R) antipode, wherein the ratio of (-)-(S)-to (+)-(R)-enantiomers is in excess of 2:1. More preferred is a mixture wherein the ratio of (-)-(S)- to (+)-(R)-enantiomers is in excess of 5:1. Most preferred is the mixture wherein the ratio of (-)-(S)- to (+)-(R)-enantiomers is 99:1 or greater.

Further, the present invention relates to a process for the preparation of a compound of formula II comprising the steps of:
(i) dissolving a racemic compound of the formula I: 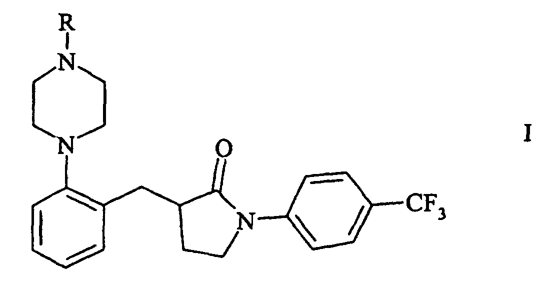 wherein R is CH₃, in an appropriate solvent in the presence of (+)-di-p-toluoyl-D-tartaric acid;
(ii) collecting the solid precipitate comprising the salt of formula III: 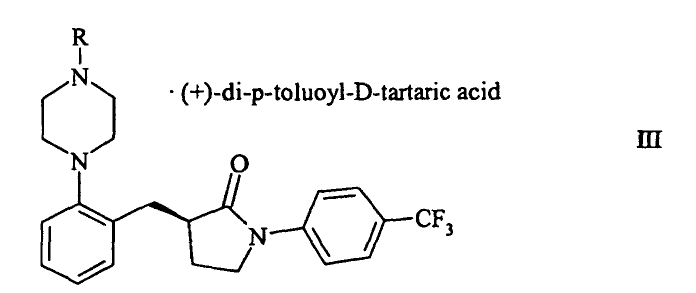 wherein R is CH₃, and (iii) treating said precipitate with a base.

Another preferred method of the invention is where the solvent in step (i) is 2-butanone, acetone, 3-pentanone, methanol, ethanol, isopropanol, and ethyl acetate, most preferably 2-butanone. Another preferred method is where the base in step (iii) is an aqueous inorganic base selected from the group consisting of sodium hydroxide, sodium carbonate, sodium bicarbonate, potassium hydroxide, potassium carbonate, potassium bicarbonate, lithium hydroxide, and ammonium hydroxide. One of skill in the art would be aware of bases appropriate for performing this particular step. Particularly preferred here are sodium or potassium hydroxide. The present invention also relates to the acid addition salts of compounds of formula III formed as intermediates in the method described above.

The present invention also relates to the pharmaceutically acceptable acid addition salts of the (-)-enantiomer of a compound of formula I. The acids which are used to prepare the pharmaceutically acceptable acid addition salts of the aforementioned base compounds of this invention are those which form non-toxic acid addition salts, *i.e.,* salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [*i*.*e*., 1,1-methylene-bis- (2-hydroxy-3-naphthoate)] salts.

This invention is also directed to isotopically-labeled compounds identical to those recited in formula II, and pharmaceutically acceptable salts thereof, but for the fact that one or more atoms are replaced therein by an atom having an atomic mass or mass number different from the an atomic mass or mass number usually found in nature. Examples of isotopes that can be incorporated into compounds of this invention include isotopes of hydrogen, carbon, nitrogen, oxygen, phosphorus, fluorine and chlorine, such as ²H, ³H, ¹¹C, ¹³C, ¹⁴C, ¹⁵N, ¹⁸O, ¹⁷O and ¹⁸F, respectively. Compounds of the present invention, prodrugs thereof, and pharmaceutically acceptable salts of said compounds, or of said prodrugs, which contain the aforementioned isotopes and/or other isotopes of other atoms are within the scope of this invention.

Certain isotopically-labeled compounds of the present invention, for example those into which radioactive isotopes such as ¹¹C, ³H and ¹⁸F are incorporated, are useful, for example, in drug and/or substrate tissue distribution assays, in particular, as a diagnostic agent to identify and localize serotonin receptors of the 5-HT1D receptor subtype. Tritiated (ie ³H), carbon-11 (ie ¹¹C) and ¹⁸F isotopes are particularly preferred for their ease of preparation and detectability. In this respect, such compounds may also be useful for assessing the density of said receptors within various regions of the central nervous systems as well as the percentage of receptor occupancy achieved using a given concentration of the compounds. Such information would be useful in establishing a dose or dose range for these compounds. Furthermore, these isotopically-labeled compounds may be used in this respect to characterize heretofore undiagnosed diseases.

Furthermore, substitution with heavier isotopes such as deuterium, ie ²H, can afford certain therapeutic advantages resulting from greater metabolic stability, for example increased in vivo half-life or reduced dosage requirements and, hence, may be preferred in some circumstances. Isotopically labeled compounds of formula II of this invention and prodrugs thereof can generally be prepared by carrying out the procedures set forth below, by substituting a readily available isotopically-labeled reagent for a non-isotopically-labeled reagent.

A particularly preferred isotopically-labeled compound of the present invention is the compound of formula II: 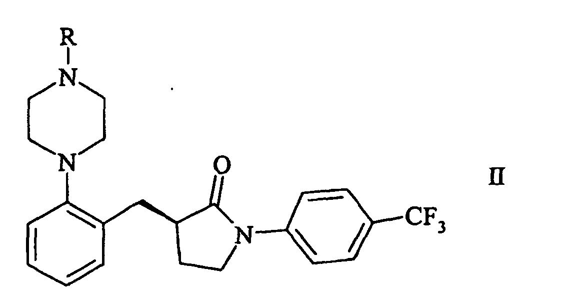 wherein R is -C³H₃. Another preferred isotopically labeled compound of the invention is one in which at least one of the fluorine atoms is ¹⁸F.

The present invention further comprises a method for the determination of the distribution in a tissue of the compound of formula II comprising the step of incubating a compound of formula II wherein one or more of the hydrogen atoms is ³H, or one or more of the fluorine atoms is ¹⁸F; or one or more of the carbon atoms is ¹¹C, or any combination thereof.

The present invention also relates to pharmaceutical compositions comprising a compound of formula II, or a pharmaceutically acceptable salt thereof. The present invention also relates to a pharmaceutical composition comprising a compound of formula II or a pharmaceutically acceptable salt thereof and a pharmaceutically acceptable carrier.

The present invention further relates to a compound of formula II, or a pharmaceutically acceptable salt or hydrated salt thereof, for use as a medicament, preferably, the use of said compound in the manufacture of a medicament for the treatment of a disease selected from the group consisting of depression, OCD and a disease, disorder or condition of the central nervous system.

The most preferred method of the present invention relates to the use of a compound of formula II or a pharmaceutically acceptable salt thereof in the manufacture of a medicament for treating a disease, disorder or condition selected from depression, generalized anxiety disorder, phobias (*e.g*., agoraphobia, social phobia and simple phobias), post-traumatic stress syndrome, avoidant personality disorder, premature ejaculation, eating disorders (*e*.*g*., anorexia nervosa and bulimia nervosa), obesity, chemical dependencies (*e.g.*, addictions to alcohol, cocaine, heroin, phenobarbital, nicotine and benzodiazepines), Alzheimer's disease, obsessive-compulsive disorder, panic disorder, memory disorders (*e.g*., dementia, amnestic disorders, and age associated memory impairment), Parkinson's diseases (*e.g*., dementia in Parkinson's disease, neuroleptic-induced parkinsonism and tardive dyskinesias), endocrine disorders (*e.g.,* hyperprolactinaemia), and gastrointestinal tract disorders involving changes in motility and secretion, in a mammal.

The present invention also relates to the use of a compound of formula II or a pharmaceutically acceptable salt thereof in the manufacture of a medicament for treating a disease, disorder or condition that can be treated by enhancing serotonergic neurotransmission in a mammal, preferably a human.

The present invention additionally relates to a pharmaceutical composition for treating a disease, disorder or condition that can be treated by enhancing serotonergic neurotransmission in a mammal, preferably a human, comprising:
a) a pharmaceutically acceptable carrier;
b) a compound of formula II or a pharmaceutically acceptable salt thereof; and
c) a 5-HT re-uptake inhibitor, preferably sertraline, or a pharmaceutically acceptable salt thereof;
wherein the amount of the active compounds (*i*.*e*., the compound of formula II and the 5-HT re-uptake inhibitor) are such that the combination is effective in treating such disease, disorder or condition.

The present invention also relates to the use of a compound of formula II, defined above, or a pharmaceutically acceptable salt thereof, and a 5-HT re-uptake inhibitor, preferably sertraline, or a pharmaceutically acceptable salt thereof, in the manufacture of a medicament for treating a disease, disorder or condition that can be treated by enhancing serotonergic neurotransmission in a mammal, preferably a human, wherein the amounts of the active compounds (i.e., the (-)-enantiomer of a compound of formula I and a 5-HT re-uptake inhibitor) are such that the combination is effective in treating such disease, disorder or condition.

The present invention also relates to the use of a 5-HT_{1A}, antagonist or a pharmaceutically acceptable salt thereof; and a compound of formula II, defined above, or a pharmaceutically acceptable salt thereof, in the manufacture of a medicament for treating a disease, disorder or condition that can be treated by enhancing serotonergic neurotransmission in a mammal, preferably a human, wherein the amounts of each active compound (*i.e.*, the 5-HT_{1A} antagonist and the compound of formula II) are such that the combination is effective in treating such disease, disorder or condition.

The term "pharmaceutically acceptable salt," as used herein, refers to a pharmaceutically acceptable acid addition salt or hydrate thereof.

The term "treating" refers to, and includes, reversing, alleviating, inhibiting the progress of, or preventing a disease, disorder or condition, or one or more symptoms thereof; and "treatment" and "therapeutically" refer to the act of treating, as defined above.

The term "enhanced serotonergic neurotransmission," as used herein, refers to increasing or improving the neuronal process whereby serotonin is released by a pre-synaptic cell upon excitation and crosses the synapse to stimulate or inhibit a post-synaptic cell.

The term "chemical dependency," as used herein, means an abnormal craving or desire for, or an addiction to, a drug. Such drugs are generally administered to the affected individual by any of a variety of means of administration, including oral, parenteral, nasal or by inhalation. Examples of chemical dependencies treatable by the methods of the present invention are dependencies on alcohol, nicotine, cocaine, heroin, phenobarbital, and benzodiazepines (e.g., Valium™). "Treating a chemical dependency," as used herein, means reducing or alleviating such dependency.

Sertraline, (1S)-*cis*-4-(3,4-dichlorophenyl)-1,2,3,4-tetrahydro-N-methyl-1-naphthalenamine, as used herein has the chemical formula C₁₇H₁₇NCl₂, and the structural formula IV:

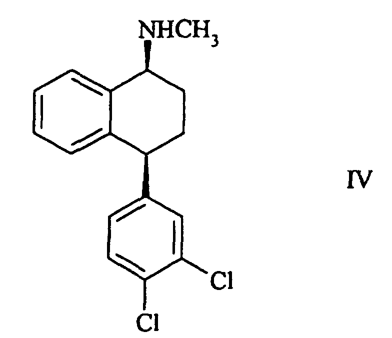

The synthesis of sertraline is described in United States Patent 4,536,518. Sertraline is useful as an antidepressant and anorectic agent, as well as in the treatment of chemical dependencies, anxiety, obsessive-compulsive disorder, depression, phobias, panic disorder, post traumatic stress disorder, and premature ejaculation.

### DESCRIPTION OF THE INVENTION

The compounds of the invention may be prepared in accordance with the reaction schemes and discussion set forth below. Unless otherwise indicated, the substituent R is as defined above. The (-)-enantiomers of the compounds of formula I (ie the compounds of formula II) have (*S*)-stereochemistry at the 3-position of the pyrrolidinone ring, and may be prepared via the separation of enantiomers in the manner described below.

The racemate of formula I, wherein R is CH₃, may be prepared in accordance with the process of Scheme I.

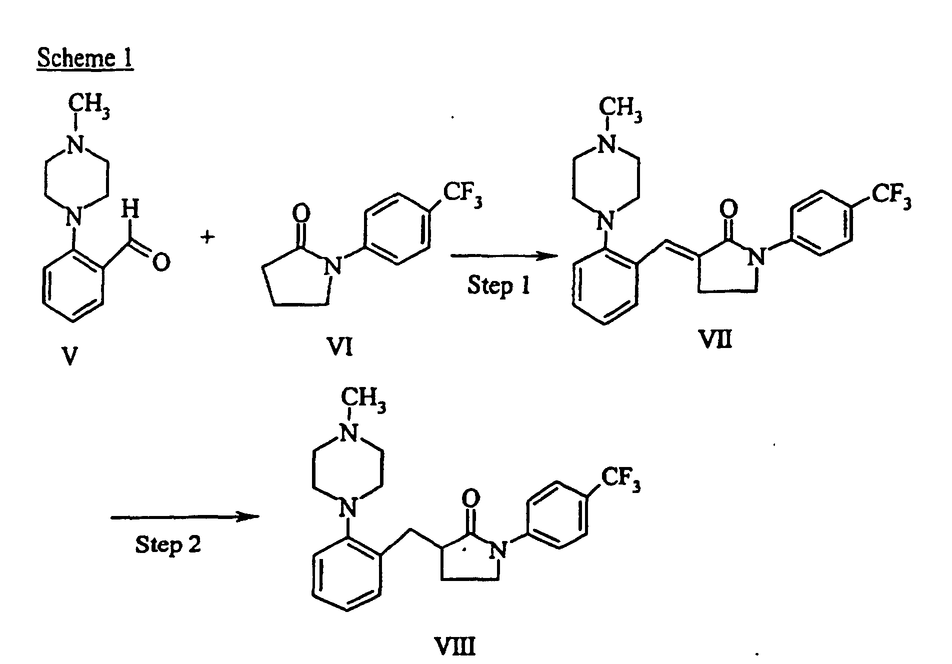

In the first step of Scheme 1, a compound of the formula VII is prepared by an aldol condensation comprising reacting a benzaldehyde of formula V with a compound of the formula VI under any conditions which favor an aldol condensation reaction. This particular condensation reaction may, *e.g.,* be carried out using any one of a variety of bases including, but not limited to, Na₂CO₃, K₂CO₃, NaH, sodium bis(trimethylsilyl)amide, potassium bis(trimethylsilyl)amide, lithium bis(trimethylsilyl)amide, pyrrolidine or piperidine, preferably NaH, in any one of a variety of solvents including, but not limited to, dimethylsulfoxide (DMSO), N,N-dimethylformamide (DMF), tetrahydrofuran (THF), methanol or ethanol, preferably DMF. The condensation reaction may be conducted at temperature in the range of about -25 °C to about 80 °C, preferably 20 to 45 °C.

The starting material of formula V, used in the first step of this procedure, may be prepared using the procedures of W. Nijhuis et al., *Synthesis,* pp. 641-645 (1987) or J. Watthey et al., *Journal of Medicinal Chemistry,* **26**, 1116-1122 (1983). The starting material of formula VI is commercially available or may be prepared according to the procedures of W.C. Shakespeare, *Tetrahedron Letters,* 1999, **40**, pp. 2035-2038, or that of T. Yamamoto and Y. Kurata, *Chemistry and Industry,* **1982**, pp. 737-738.

In the process of reacting compounds of formulae V and VI to give VII, an intermediate compound of formula IX: 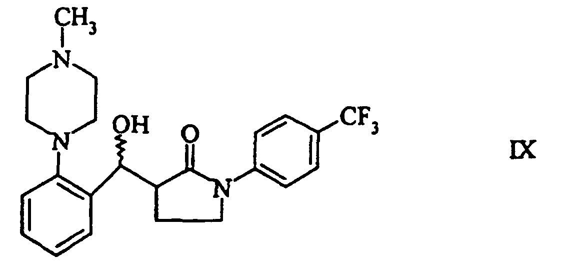 is formed and may be identified using one or more analytical techniques including thin layer chromatography (tlc) and mass spectrometry (MS):

In some instances, it may be desirable to isolate the intermediate of formula IX. This intermediate alcohol of formula IX may then be subjected to conditions under which a water molecule is eliminated to produce the compound of formula VII. The dehydration of a compound of formula IX may be carried out by, *e.g*., dissolving the compound of formula IX in a suitable inert solvent, such as benzene, xylene, etc., and heating the solution to the solvent reflux temperature in the presence of a catalytic amount of benzene- or toluene-sulfonic acid with some provision for physical or chemical removal of the water generated. Such water removal techniques may include, *e.g*., the use of molecular sieves or a Dean-Stark trap to isolate the water created as a water-solvent azeotrope. A detailed description of the aldol condensation reaction process may be found in Herbert O. House, Modem Synthetic Reactions, 2^{nd} Edition, pp. 629-682 (W. A. Benjamin, Menlo Park, CA, 1972).

Conversion of the compound of formula VII to the compound of formula VIII involves the reduction of the α-benzylidenyl carbon-carbon double bond, which may be accomplished using any one of several methods available to one skilled in the art. For example, the reduction of the carbon-carbon double bond of VII may be effected via a hydrogenation reaction using hydrogen gas (H2) in the presence of catalysts such as, *e*.*g*., Pd/C, Pd/Ba₂SO₄, Pt/C, tris-(triphenylphosphine) rhodium chloride (Wilkinson's catalyst), in solvents such as, *e.g*., methanol, ethanol, THF, dioxane or ethyl acetate, at pressures of from 1-5 atmospheres and at temperatures from about 10 °C to about 60 °C. Hydrogenation techniques such as this are familiar to those of skill in the art, as described in Paul Rylander, Catalytic Hydrogenation in Organic Synthesis, pp. 31-63 (Academic Press Inc., San Diego, 1979). Preferred conditions for this hydrogenation are Pd on carbon in methanol at 25 °C and 50 psi of H₂ pressure. This method also provides means for the introduction of hydrogen isotopes (*i.e*., deuterium and tritium) by replacing ¹H₂ with ²H₂ or ³H₂ in the procedure.

Alternatively, the reduction of the carbon-carbon double bond of the compound of formula VII to produce a compound of formula VIII may be carried out via an alternative procedure which employs reagents such as ammonium formate and Pd/C in methanol at reflux temperature under an inert atmosphere (*e.g*., nitrogen or argon). Another method of reducing the double bond involves reacting a compound of formula VII with samarium iodide (SmI2) in methanol or ethanol at room temperature, as described by R. Yanada et al., *Synlett*, **1995**, 443-444.

The compound of the formula VII may be generated in one of two geometric isomer forms, E- (entgegen) or Z- (zusammen), depending upon the aldol condensation conditions employed, and as such the compound of formula VII may be isolated as a single isomer or as a mixture of the two forms. Nonetheless, subsequent reduction of the carbon-carbon double bond of either form, or a mixture thereof, will produce the compound of formula VIII. The compound of formula VIII, which contains a chiral carbon, may exist in either the (R)- or (S)-enantiomer form, or as a mixture or racemate of the two forms. These enantiomers are separable using one or more techniques available to one skilled in the art. These include, for example: chromatographic separation of the racemic compound using a chiral stationary phase, (*e.g*., one of the commercially available cyclodextrins); derivatization using a chiral reagent that creates a covalent bond with the enantiomers, followed by separation of the diastereomers thus produced and removal of the derivatizing portion, to regenerate the individual enantiomer(s); and chemical resolution using an enantiomerically pure resolving agent to form a pair of diastereomeric salts which, by virtue of their different physical properties, can be separated (*e.g*., by selective recrystallization), followed by isolation of the purified enantiomer from the salt.

In the process of the present invention, the compound of formula VIII or 3-[[2-(4-methyl-1-piperazinyl)phenyl]methyl]-1-[4-(trifluoromethyl)phenyl]-2-pyrrolidinone is resolved via selective crystallization via the use of either (+)-di-*p*-toluoyl-D-tartaric acid or (-)-di-p-toluoyl-L-tartaric acid. The compound of formula VIII is reacted with the di-p-toluoyl-tartaric acid in a suitable solvent, including but not limited to 2-butanone, acetone, 3-pentanone, methanol, ethanol, isopropanol, and ethyl acetate, preferably 2-butanone. Depending on which tartaric acid is employed, only one of the diastereomeric di-p-toluoyl tartrate salts precipitates, i.e., when the (+)-di-p-toluoyl-D-tartaric acid salt of the racemate is formed, the 3-(R)-enantiomer tartrate remains in solution and the tartrate salt of the 3-(S)-enantiomer precipitates, *i.e.,* the compound of formula III, and when the (-)-di-*p*-toluoyl-L-tartaric acid salt is formed, the 3-(S)-enantiomer tartrate remains in solution and the tartrate salt of the 3-(R)-enantiomer precipitates. If the tartrate salt of the desired enantiomer remains in solution, it may be recovered by evaporation of the solvent.

In the preferred embodiment of the method of the invention, 3-[[2-(4-methyl-1-piperazinyl)phenyl]methyl]-1-[4-(trifluoromethyl)phenyl]-2-pyrrolidinone is resolved using (+)-di-p-toluoyl-D-tartaric acid which produces a salt with an enantiomeric excess of the (-)-enantiomer of 3-[[2-(4-methyl-1-piperazinyl)phenyl]methyl]-1-[4-(trifluoromethyl)phenyl]-2-pyrrolidinone of at least 80%, and yields after treatment with base an enantiomer with an optical purity of at least 96%. The enantiomeric excess is evaluated using methods known in the art, such as., *e.g*., the method disclosed in J. Jacques, et al., Enantiomers, Racemates and Resolutions (John Wiley & Sons, New York, 1981).

The resolution of 3-[[2-(4-methyl-1-piperazinyl)phenyl]methyl]-1-[4-(trifluoromethyl)phenyl]-2-pyrrolidinone to obtain the (-)-enantiomer is illustrated in Scheme 2.

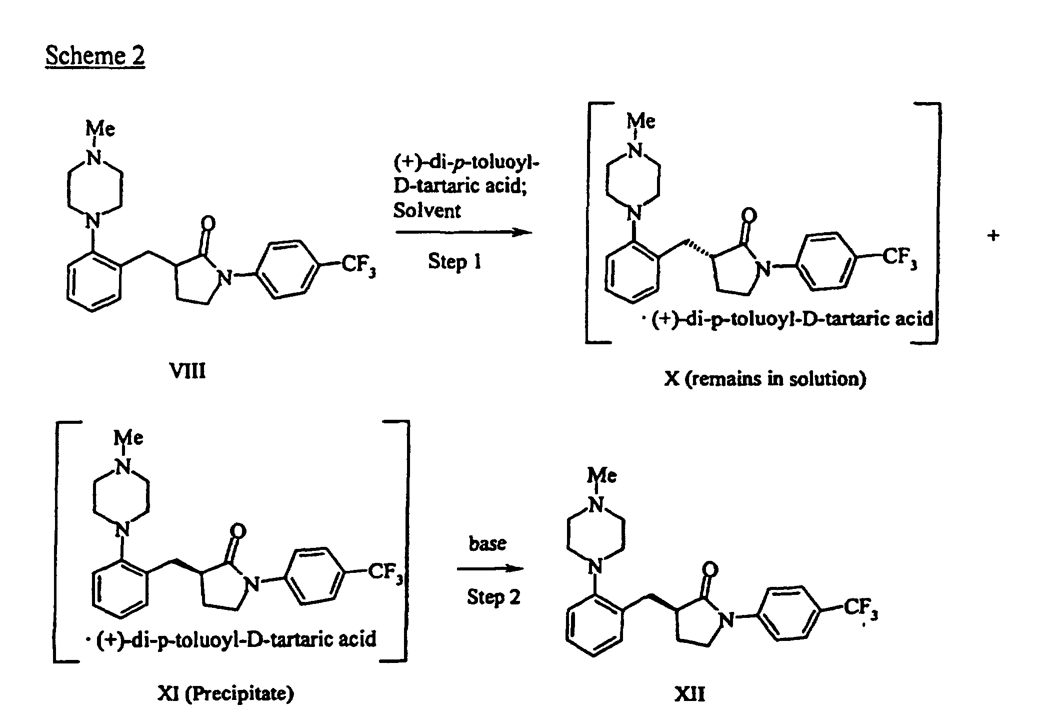

Step 1 in Scheme 2 is the resolution of racemic compound of formula VIII. The racemate of formula VIII is dissolved in a solvent, such as 2-butanone, acetone, 3-pentanone, methanol, ethanol, isopropanol, and ethyl acetate, preferably 2-butanone, at a temperature between room temperature and 80 °C, preferably about 50 °C, treated with 0.5 to 1.25 equivalents of (+)-di-*p*-toluoyl-D-tartaric acid, preferably about one equivalent, cooled to room temperature, and stirred for a period of 5 to 48 hours, preferably about 15 hours, which affords a precipitate salt of formula XI, which is collected by filtration from the solvent. The salt of formula X remains in the mother liquor.

Step 2 in Scheme 2 is the conversion of the salt of formula XI to the free base. The salt of formula XI is treated with an aqueous inorganic base, such that the pH is greater than 8, such as, e.g., sodium hydroxide, sodium carbonate, sodium bicarbonate, potassium hydroxide, potassium carbonate, potassium bicarbonate, lithium hydroxide, ammonium hydroxide, preferably, sodium or potassium hydroxide, in a solvent such as dichloromethane, toluene, diisopropyl ether, methyl *tert*-butyl ether, preferably dichloromethane. The organic layer is concentrated to afford a compound of formula XII.

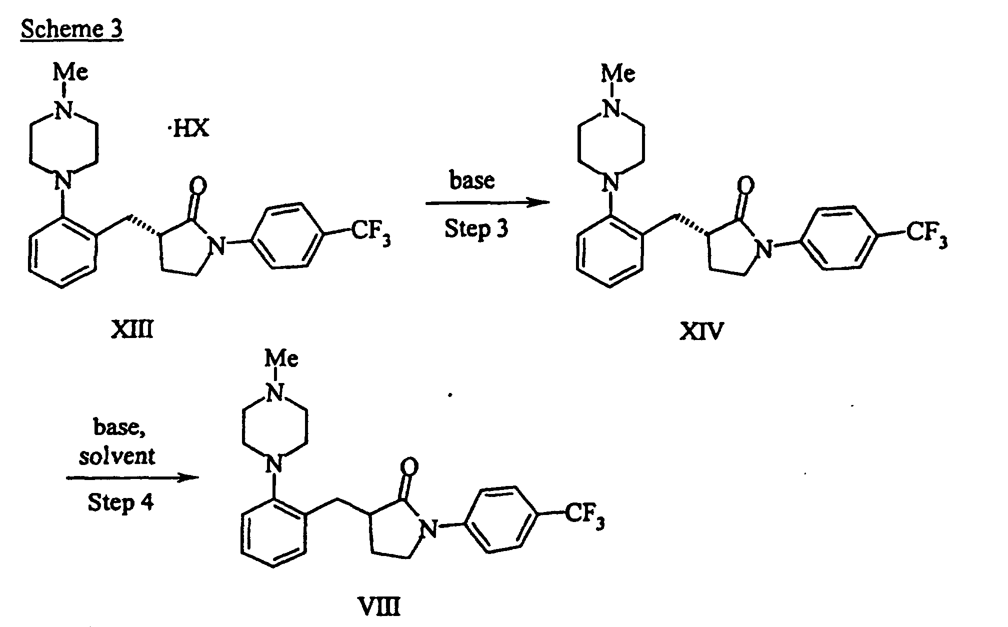

Step 3 in Scheme 3 is the generation of a free base, XIV, from a salt of formula XIII, and is applicable in general to salts obtained via the methods of the invention, *e.g*., the salt of formula X remaining in solution after the reaction of (+)-di-p-toluoyl-D-tartaric acid with the racemate of formula VIII. The salt of formula XIII is treated with an aqueous inorganic base, such that the pH is greater than 8, in a solvent such as dichloromethane, toluene, diisopropyl ether, methyl *tert*-butyl ether, preferably dichloromethane. The organic layer is concentrated to afford the (+)-enantiomer of formula XIV.

Step 4 in Scheme 3 is a racemization of the (+)-enantiomer of formula XIV. A compound of formula XIV is treated with a base such as potassium t-butoxide, sodium methoxide, potassium methoxide, potassium ethoxide, sodium ethoxide, sodium isopropoxide, potassium isopropoxide, in either a catalytic amount or greater amount, preferably 0.2 equivalent, at a temperature above 50 °C, preferably at about 65 °C, for a period of time between 3 and 24 hours, preferably 12 hours to afford a compound of formula VIII which is a racemate.

Unless indicated otherwise, the pressure under which each of the above reactions is conducted is not critical. Generally, the reactions will be conducted at a pressure of about one to about three atmospheres, preferably at ambient pressure (about one atmosphere).

The compounds of the invention are basic in nature and are capable of forming a wide variety of different salts with various inorganic and organic acids. Although such salts must be pharmaceutically acceptable for administration to animals, it is often desirable in practice to initially isolate the compound of formula II from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent such as methanol or ethanol. Upon careful evaporation of the solvent, the desired solid salt is obtained.

The acids used to prepare the pharmaceutically acceptable acid addition salts of the base compounds of this invention are those which form non-toxic acid addition salts, *i.e*., salts containing pharmacologically acceptable anions, such as hydrochloride, hydrobromide, hydroiodide, nitrate, sulfate or bisulfate, phosphate or acid phosphate, acetate, lactate, citrate or acid citrate, tartrate or bitartrate, succinate, maleate, fumarate, gluconate, saccharate, benzoate, methanesulfonate and pamoate [*i*.*e*., 1,1'-methylene-bis-(2-hydroxy-3-naphthoate)] salts.

The (-)-enantiomers of the compounds of formula I and their pharmaceutically acceptable salts (hereinafter also referred to, collectively, as "the active compounds") are useful psychotherapeutics and are potent selective antagonists of the serotonin 1D (5-HT_{1D}) receptor. The active compounds are useful in the treatment of depression, generalized anxiety disorder, phobias (*e.g*., agoraphobia, social phobia and simple phobias), post-traumatic stress syndrome, avoidant personality disorder, sexual dysfunction (*e.g*., premature ejaculation), eating disorders (*e.g*., anorexia nervosa and bulimia nervosa), obesity, chemical dependencies (*e.g*., addictions to alcohol, cocaine, heroin, phenobarbital, nicotine and benzodiazepines), Alzheimer's disease, obsessive-compulsive disorder, panic disorder, memory disorders (*e.g*., dementia, amnestic disorders, and age-associated memory impairment), Parkinson's diseases (*e.g*., dementia in Parkinson's disease, neuroleptic-induced parkinsonism and tardive dyskinesias), endocrine disorders (*e.g*., hyperprolactinaemia), and gastrointestinal tract disorders involving changes in motility and secretion. These compounds are also useful as vasodilators.

Further, the compound of formula XII has been observed to metabolize to another compound of the invention of formula XV: 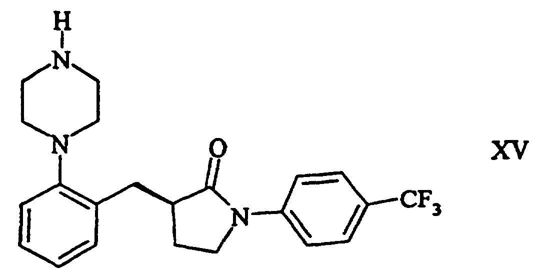 This compound also possesses potent selective 5-HT_{1D} receptor antagonist activity and may be prepared in accordance with the methods presented above.

The affinity of the compounds of this invention for the various serotonin-1 receptors can be determined using standard radioligand binding assays as described in the literature. The 5-HT_{1A} affinity can be measured using the procedure of Hoyer *et al. (Brain Res*., **376**, p. 85 (1986)). The 5-HT_{1D} affinity can be measured using the procedure of Heuring and Peroutka (*J*. *Neurosci.,* **7**, 894 (1987)).

The *in vitro* activity of the compounds of the present invention at the 5-HT_{1D} binding site may be determined according to the following procedure. Bovine caudate tissue is homogenized and suspended in 20 volumes of a buffer containing 50 mM TRIS·hydrochloride (tris[hydroxymethyl]aminomethane hydrochloride) at a pH of 7.7. The homogenate is then centrifuged at 45,000G for 10 minutes. The supernatant is then discarded and the resulting pellet resuspended In approximately 20 volumes of 50 mM TRIS·hydrochloride (HCI) buffer at pH 7.7. This suspension is then preincubated for 15 minutes at 37°C, after which the suspension is centrifuged again at 45,000G for 10 minutes and the supernatant discarded. The resulting pellet (approximately 1 gram) is resuspended in 150 ml of a buffer of 15 mM TRIS-hydrochloride (HCl) containing 0.01 percent ascorbic acid with a final pH of 7.7 and also containing 10 µM pargyline and 4 mM calcium chloride (CaCl₂). The suspension is kept on ice at least 30 minutes prior to use.

The inhibitor, control or vehicle is then incubated according to the following procedure. To 50 µl of a 20 percent dimethylsulfoxide (DMSO)/80 percent distilled water solution is added 200 µl of tritiated 5-hydroxytryptamine (2 nM) in a buffer of 50 mM TRIS·hydrochloride containing 0.01 percent ascorbic acid at pH 7.7 and also containing 10 µM pargyline and 4 µM calcium chloride, plus 100 nM of 8-hydroxy-DPAT (dipropylaminotetraline) and 100 nM of mesulergine. To this mixture is added 760 µl of bovine caudate tissue, and the resulting suspension is vortexed to ensure a homogenous suspension. The suspension is then incubated in a shaking water bath for 30 minutes at 25°C. After incubation is complete, the suspension is filtered using glass fiber filters (*e.g*., Whatman GF/B-filters™). The pellet is then washed three times with 4 ml of a buffer of 50 mM TRIS·hydrochloride at pH 7.7. The pellet is then placed in a scintillation vial with 5 ml of scintillation fluid (Aquasol 2™) and allowed to sit overnight. The percent inhibition can be calculated for each dose of the compound. An IC₅₀ value can then be calculated from the percent inhibition values.

The activity of the compounds of the present invention for 5-HT_{1A} binding ability can be determined according to the following procedure. Rat brain cortex tissue is homogenized and divided into samples of 1 gram lots and diluted with 10 volumes of 0:32 M sucrose solution. The suspension is then centrifuged at 900G for 10 minutes and the supernatant separated and recentrifuged at 70,000G tor 15 minutes. The supernatant is discarded and the pellet re-suspended in 10 volumes of 15 mM TRIS·hydrochloride at pH 7.5. The suspension is allowed to incubate for 15 minutes at 37°C. After pre-incubation is complete, the suspension is centrifuged at 70,000G for 15 minutes and the supernatant discarded. The resulting tissue pellet is resuspended in a buffer of 50mM TRIS·hydrochloride at pH 7.7 containing 4 mM of calcium chloride and 0.01 percent ascorbic acid. The tissue is stored at -70°C until ready for an experiment. The tissue can be thawed immediately prior to use, diluted with 10 µm pargyline and kept on ice.

The tissue is then incubated according to the following procedure. Fifty microliters of control, inhibitor, or vehicle (1 percent DMSO final concentration) is prepared at various dosages. To this solution is added 200µl of tritiated 8-hydroxy-2-dipropylaminotetralin (DPAT) at a concentration of 1.5 nM in a buffer of 50 mM TRIS·hydrochloride at pH 7.7 containing 4 mM calcium chloride, 0.01 percent ascorbic acid and pargyline. To this solution is then added 750 µl of tissue and the resulting suspension is vortexed to ensure homogeneity. The suspension is then incubated in a shaking water bath for 30 minutes at 37°C. The solution is then filtered, washed twice with 4 ml of 10 mM TRIS·hydrochloride at pH 7.5 containing 154 mM of sodium chloride. The percent inhibition is calculated for each dose of the compound, control or vehicle. IC₅₀ values are calculated from the percent inhibition values.

The compounds of the present invention were assayed for 5-HT_{1D} and 5-HT_{1A} affinity using the aforementioned procedures. In particular, the compound of formula XII exhibited within experimental uncertainty an IC₅₀ less than 10.0 nM for 5-HT_{1D} affinity and an IC₅₀ less than 400 nM for 5-HT_{1A} affinity.

The agonist and antagonist activities of the compounds of the invention at 5-HT_{1A} and 5-HT_{1D} receptors can be determined using a single saturating concentration according to the following procedure. Male Hartley guinea pigs are decapitated and 5-HT_{1A} receptors are dissected out of the hippocampus, while 5-HT_{1D} receptors are obtained by slicing at 350 mM on a McIlwain tissue chopper and dissecting out the substantia nigra from the appropriate slices. The individual tissues are homogenized in 5 mM HEPES buffer containing 1 mM EGTA (pH 7.5) using a hand-held glass-Teflon® homogenizer and centrifuged at 35,000 x g for 10 minutes at 4°C. The pellets are resuspended in 100 mM HEPES butter containing 1 mM EGTA (pH 7.5) to a final protein concentration of 20 mg (hippocampus) or 5 mg (substantia nigra) of protein per tube. The following agents are added so that the reaction mix in each tube contained 2.0 mM MgCl₂, 0.5 mM ATP, 1.0 mM cAMP, 0.5 mM IBMX, 10 mM phosphocreatine, 0.31 mg/mL creatine phosphokinase, 100 µM GTP and 0.5-1 microcuries a[³²P]-ATP (30 Ci/mmol: NEG-003 - New England Nuclear). Incubation is initiated by the addition of tissue to siliconized microfuge tubes (in triplicate) at 30°C for 15 minutes. Each tube receives 20 µL tissue, 10 µL drug or buffer (at 10X final concentration), 10 µL 32 nM agonist or buffer (at 10X final concentration), 20 µL forskolin (3 µM final concentration) and 40 µL of the preceding reaction mix. Incubation is terminated by the addition of 100 µL 2% SDS, 1.3 mM cAMP, 45 mM ATP solution containing 40,000 dpm [³H]-cAMP (30 Ci/mmol: NET-275 - New England Nuclear) to monitor the recovery of cAMP from the columns. The separation of [³²P]-ATP and [³²P]-cAMP is accomplished using the method of Salomon *et al*., *Analytical Biochemistry,* **58**, pp. 541-548 (1974). Radioactivity is quantified by liquid scintillation counting. Maximal inhibition is defined by 10µM (R)-8-OH-DPAT for 5-HT_{1A} receptors, and 320 nM 5-HT_{1D} for 5-HT_{1D}, receptors. Percent inhibitions by the test compounds are then calculated in relation to the inhibitory effect of (R)-8-OH-DPAT for 5-HT_{1A} receptors or 5-HT for 5-HT_{1D} receptors. The reversal of agonist induced inhibition of forskolin-stimulated adenylate cyclase activity is calculated in relation to the 32 nM agonist effect.

The compounds of the invention can be tested for *in vivo* activity for antagonism of 5-HT_{1D} agonist-induced hypothermia in guinea pigs according to the following procedure.

Male Hartley guinea pigs from Charles River, weighing 250-275 grams on arrival and 300-600 grams at testing, serve as subjects in the experiment. The guinea pigs are housed under standard laboratory conditions on a 7 a.m. to 7 p.m. lighting schedule for at least seven days prior to experimentation. Food and water are available *ad libitum* until the time of testing.

The compounds of the invention can be administered as solutions in a volume of 1 ml/kg. The vehicle used is varied depending on compound solubility. Test compounds are typically administered either sixty minutes orally (p.o.) or 0 minutes subcutaneous (s.c.) prior to the 5-HT_{1D} agonist, [3-(1-methylpyrrolidin-2-ylmethyl)-1H-indol-5-yl]-(3-nitropyridin-2-yl)-amine, which is administered at a dose of 5.6 mg/kg, s.c. Before a first temperature reading is taken, each guinea pig is placed in a clear plastic shoe box containing wood chips and a metal grid floor and allowed to acclimate to the surroundings for 30 minutes. Animals are then returned to the same shoe box after each temperature reading. Prior to each temperature measurement each animal is firmly hold with one hand for a 30-second period. A digital thermometer with a small animal probe is used for temperature measurements. The probe is made of semi-flexible nylon with an epoxy tip. The temperature probe is inserted 6 cm. into the rectum and held there for 30 seconds or until a stable recording is obtained. Temperatures are then recorded.

In p.o. screening experiments, a "pre-drug" baseline temperature reading is made at -90 minutes, the test compound is given at -60 minutes and an additional -30 minute reading is taken. The 5-HT_{1D} agonist is then administered at 0 minutes and temperatures are taken 30, 60, 120 and 240 minutes later.

In subcutaneous screening experiments, a pre-drug baseline temperature reading is made at -30 minutes. The test compound and the 5-HT_{1D} agonist are given concurrently and temperatures are taken at 30, 60, 120 and 240 minutes later. Data are analyzed with two-way analysis of variants with repeated measures in Newman-Keuls *post hoc* analysis.

As noted previously, the radiolabeled compounds of the present invention, particularly wherein the molecule contains a radio-isotope such as ¹¹C, ¹⁴C, ³H or ¹⁸F, is useful as a diagnostic agent to identify and localize serotonin receptors of the 5-HT_{1D} receptor subtype. The radiolabeled compounds of the invention can also be useful for assessing the density of said receptors within various regions of the central nervous system as well as the percentage of receptor occupancy achieved using a given concentration of the compounds. The diagnostic use of these labeled compounds in techniques, such as positron emission tomography (PET), among others, may be carried out analogous to those set forth in Ginovart *et al., Synapse,* **35**:192-200 (2000); Shiue *et al., Synapse,* **25**:147-154 (1997); Drevets *et al*., *Biol*. *Psychiatry,* **46**:1375-1387 (1999); Hall *et al*., *Brain Research,* **745**:96-108 (1997); Pike *et al*., *Eur. J. Pharm.,* **301**:R5-R7 (1996); and Koepp *et al*., *Nature,* **393:**266-268. Information obtained from such radiolabeled compound assays is useful in establishing a dose or dose range for the compounds as therapeutic agents. Furthermore, these radiolabeled compounds may be used in this respect to characterize heretofore undiagnosed diseases.

A determination of the binding to 5-HT_{1D} receptors in bovine membranes used in conjunction with the radiolabeled compounds may be carried out as follows: Bovine brain corpus striata are dissected out and homogenized with a Kinematica tissue homogenizer (Kinematica Inc., Cincinnati, OH), using 40 volumes of 50 mM TRIS HCl [tris(hydroxymethyl)aminomethane hydrochloride] per gram of tissue at pH 7.7. The homogenate is centrifuged at 40,000 x G for 10 min, the resulting pellet is re-suspended in fresh buffer and incubated for 20 minutes at 37 °C. The homogenate is again centrifuged at 40,000 x G. The final pellet (1 gram) is re-suspended in 10 ml of TRIS HCl containing 1 mg/ml of ascorbic acid, 4 mM CaCl₂ and 10 µM pargyline at pH 7.7. The binding is carried out in 0.75 ml, 96 well plates; each well containing 50 µl of a test compound or solvent, 400 µl of the [³H]-labeled compound of Example 9 (2 nM final concentration) and 50 µl of membranes. Nonspecific binding is determined in the presence of 10 µM 5-HT. The tubes are incubated for 30 minutes at 37 °C, filtered onto GF/B filters using a Brandel cell harvester (Brandel, 8561 Atlas Drive, Gaithersburg, MD 20877) and washed with 3 x 0.5 ml of 50 mM TRIS HCl at pH 7.7. The filters are counted using a Wallac Betaplate® liquid scintillation counter (PerkinElmer Life Sciences, Wallac Inc., 9238 Gaither Rd., Gaithersburg, Maryland 20877). The percent inhibition of total specific binding is calculated and plotted for each inihibitor concentration and the IC50 is calculated from this data.

The compounds of the invention may advantageously be used in conjunction with one or more other therapeutic agents, for instance, different antidepressant agents such as tricyclic antidepressants (*e.g*., amitriptyline, dothiepin, doxepin, trimipramine, butripyline, clomipramine, desipramine, imipramine, iprindole, lofepramine, nortriptyline or protriptyline), monoamine oxidase inhibitors (*e.g*., isocarboxazid, phenelzine or tranylcyclopramine) or 5-HT re-uptake inhibitors (*e.g*., fluvoxamine, sertraline, fluoxetine or paroxetine), and/or with antiparkinsonian agents such as dopaminergic antiparkinsonian agents (*e.g*., levodopa, preferably in combination with a peripheral decarboxylase inhibitor, *e.g*., benserazide or carbidopa, or with a dopamine agonist, *e.g.,* bromocriptine, lysuride or pergolide). It is to be understood that the present invention covers the use of a compound of general formula II or a physiologically acceptable salt or solvate thereof in combination with one or more other therapeutic agents.

The compound of formula II, or a pharmaceutically acceptable salt thereof, in combination with a 5-HT re-uptake inhibitor (*e.g*., fluvoxamine, sertraline, fluoxetine or paroxetine), preferably sertraline, or a pharmaceutically acceptable salt or polymorph thereof, (the combination of the compound of formula II with a 5-HT re-uptake inhibitor is referred herein to as "the active combination"), are useful psychotherapeutics and may be used in the treatment of disease, disorder or condition the treatment of which is facilitated by enhanced serotonergic neurotransmission (*e.g*., depression, generalized anxiety disorder, phobias, post-traumatic stress syndrome, avoidant personality disorder, sexual dysfunction, eating disorders, obesity, chemical dependencies, Alzheimer's disease, obsessive-compulsive disorder, panic disorder, memory disorders (*e.g*., dementia, amnestic disorders, and age-associated memory impairment), Parkinson's diseases (*e.g*., dementia in Parkinson's disease, neuroleptic-induced Parkinsonism and tardive dyskinesias), endocrine disorders (*e.g*., hyperprolactinaemia), and gastrointestinal tract disorders involving changes in motility and secretion.

Serotonin (5-HT) re-uptake inhibitors, preferably sertraline, exhibit positive activity against depression; chemical dependencies; anxiety disorders including panic disorder, generalized anxiety disorder, agoraphobia, simple phobias, social phobia, and post-traumatic stress disorder; obsessive-compulsive disorder; avoidant personality disorder and premature ejaculation in mammals, including humans, due in part to their ability to block the synaptosomal uptake of serotonin.

United States Patent 4,536,518 describes the synthesis, pharmaceutical composition and use of sertraline for depression and is hereby incorporated by reference in its entirety.

Activity of the active combination as antidepressants and related pharmacological properties can be determined by methods (1)-(4) below, which are described in Koe, B. *et al*., *J. Pharmacal. Exper. Ther.,* **226**(3), pp. 686-700 (1983). Specifically, activity can be determined by studying (1) their ability to affect the efforts of mice to escape from a swim-tank (Porsolt mouse "behavior despair" test), (2) their ability to potentiate 5-hydroxytryptophan-induced behavioral symptoms in mice in vivo, (3) their ability to antagonize the serotonin-depleting activity of p-chloroamphetamine hydrochloride in rat brain *in vivo,* and (4) their ability to block the uptake of serotonin, norepinephrine and dopamine by synaptosomal rat brain calls *in vitro.* The ability of the active combination to counteract reserpine hypothennia in mice *in vivo* can be determined according to the methods described in U.S. Pat. No. 4,029,731.

The compositions of the present invention may be formulated in a conventional manner using one or more pharmaceutically acceptable carriers. Thus, the active compounds of the invention may be formulated for oral, buccal, intranasal, parenteral (*e.g*., intravenous, intramuscular or subcutaneous) or rectal administration or in a form suitable for administration by inhalation or insufflation.

For oral administration, the pharmaceutical compositions may take the form of, for example, tablets or capsules prepared by conventional means with pharmaceutically acceptable excipients such as binding agents (*e.g*., pregelatinised maize starch, polyvinylpyrrolidone or hydroxypropyl methylcellulose); fillers (*e.g*., lactose, microcrystalline cellulose or calcium phosphate); lubricants (*e.g*., magnesium stearate, talc or silica); disintegrants (*e.g*., potato starch or sodium starch glycolate); or wetting agents (*e.g*., sodium lauryl sulfate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, methyl cellulose or hydrogenated edible fats); emulsifying agents (*e.g*., lecithin or acacia); non-aqueous vehicles (*e.g*., almond oil, oily esters or ethyl alcohol); and preservatives (*e.g*., methyl or propyl p-hydroxybenzoates or sorbic acid).

For buccal administration, the composition may take the form of tablets or lozenges formulated in conventional manner.

The active compounds of the invention may be formulated for parenteral administration by injection, including using conventional catheterization techniques or infusion. Formulations for injection may be presented in unit dosage form, *e.g*., in ampoules or in multi-dose containers, with an added preservative. The compositions may take such forms as suspensions, solutions or emulsions in oily or aqueous vehicles, and may contain formulating agents such as suspending, stabilizing and/or dispersing agents. Alternatively, the active ingredient may be in powder form for reconstitution with a suitable vehicle, *e.g*., sterile pyrogen-free water, before use.

The active compounds of the invention may also be formulated in rectal compositions such as suppositories or retention enemas, *e.g*., containing conventional suppository bases such as cocoa butter or other glycerides.

For intranasal administration or administration by inhalation, the active compounds of the invention are conveniently delivered in the form of a solution or suspension from a pump spray container that is squeezed or pumped by the patient or as an aerosol spray presentation from a pressurized container or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

A proposed dose of the active compounds of the invention for oral, parenteral or buccal administration to the average adult human for the treatment of the conditions referred to above (*e.g*., depression) is 0.1 to 200 mg of the active ingredient per unit dose which could be administered, for example, 1 to 4 times per day.

Aerosol formulations for treatment of the conditions referred to above (*e.g*., depression) in the average adult human are preferably arranged so that each metered dose or "puff", of aerosol contains 20 µg to 1000 µg of the compound of the invention. The overall daily dose with an aerosol will be within the range 100 µg to 10 mg. Administration may be several times daily, for example 2, 3, 4 or 8 times, giving for example, 1, 2 or 3 doses each time.

In connection with the use of an active compound of this invention with a 5-HT re-uptake inhibitor, preferably sertraline, for the treatment of subjects possessing any of the above conditions, these compounds may be administered either alone or in combination with pharmaceutically acceptable carriers by any of the routes previously indicated, and that such administration can be carried out in both single and multiple dosages. More particularly, the active combination can be administered in a wide variety of different dosage forms, *i.e*., they may be combined with various pharmaceutically-acceptable inert carriers in the form of tablets, capsules, lozenges, troches, hard candies, powders, sprays, aqueous suspension, injectable solutions, elixirs, syrups, and the like. Such carriers include solid diluents or fillers, sterile aqueous media and various non-toxic organic solvents, etc. Moreover, such oral pharmaceutical formulations can be suitably sweetened and/or flavored by means of various agents of the type commonly employed for such purposes. In general, the active compounds of the invention are present in such dosage forms at concentration levels ranging from about 0.5% to about 90% by weight of the total composition, *i.e.,* in amounts which are sufficient to provide the desired unit dosage, and a 5-HT re-uptake inhibitor, preferably sertraline, is present in such dosage forms at concentration levels ranging from about 0.5% to about 90% by weight of the total composition, *i.e.,* in amounts which are sufficient to provide the desired unit dosage.

A proposed daily dose of an active compound of this invention in the combination formulation (a formulation containing an active compound of this invention and a 5-HT re-uptake inhibitor) for oral, parenteral, rectal or buccal administration to the average adult human for the treatment of the conditions referred to above is from about 0.01 mg to about 2000 mg, preferably from about 0.1 mg to about 200 mg of the active compound of the invention per unit dose which could be administered, for example, 1 to 4 times per day.

A proposed daily dose of a 5-HT re-uptake inhibitor, preferably sertraline, in the combination formulation for oral, parenteral or buccal administration to the average adult human for the treatment of the conditions referred to above is from about 0.1 mg to about 2000 mg, preferably from about 1 mg to about 200 mg of the 5-HT re-uptake inhibitor per unit dose which could be administered, for example, 1 to 4 times per day.

A preferred dose ratio of sertraline to an active compound of this invention in the combination formulation for oral, parenteral or buccal administration to the average adult human for the treatment of the conditions referred to above is from about 0.00005 to about 20,000, preferably from about 0.25 to about 2,000.

Aerosol combination formulations for treatment of the conditions referred to above in the average adult human are preferably arranged so that each metered dose or "puff" of aerosol contains from about 0.01 µg to about 1000 µg of the active compound of this invention, preferably from about 1 µg to about 10 mg of such compound. Administration may be several times daily, for example 2, 3, 4 or 8 times, giving for example, 1, 2 or 3 doses each time.

Aerosol formulations for treatment of the conditions referred to above in the average adult human are preferably arranged so that each metered dose or "puff" of aerosol contains from about 0.01 mg to about 2000 mg of a 5-HT re-uptake inhibitor, preferably sertraline, preferably from about 1 mg to about 200 mg of sertraline. Administration may be several times daily, for example 2, 3, 4 or 8 times, giving for example, 1, 2 or 3 doses each time.

As previously indicated, a 5-HT re-uptake inhibitor, preferably sertraline, in combination with a compound of formula II, is readily adapted to therapeutic use as antidepressant agents. In general, these antidepressant compositions containing a 5-HT re-uptake inhibitor, preferably sertraline, and the compound of formula II are normally administered in dosages ranging from about 0.01 mg to about 100 mg per kg of body weight per day of a 5-HT re-uptake inhibitor, preferably sertraline, preferably from about 0.1 mg. to about 10 mg per kg of body weight per day of sertraline; with from about 0.001 mg. to about 100 mg per kg of body weight per day of the (-)-enantiomer of a compound of formula II, preferably from about 0.01 mg to about 10 mg per kg of body weight per day of said (-)-enantiomer, although variations will necessarily occur depending upon the conditions of the subject being treated and the particular route of administration chosen.

The following examples illustrate the preparation of the compounds of the present invention. Melting points are uncorrected. NMR data are reported in parts per million (δ) and are referenced to the deuterium lock signal from the sample solvent (deuterochloroform unless otherwise specified). Specific rotations were measured at room temperature using the sodium D line (589 nm). Commercial reagents were utilized without further purification. THF refers to tetrahydrofuran. DMF refers to N,N-dimethylformamide. Room or ambient temperature refers to 20-25°C. All non-aqueous reactions were run under a nitrogen atmosphere for convenience and to maximize yields. Concentration at reduced pressure means that a rotary evaporator was used.

### EXAMPLES

### Example 1

### 2-(4-Methyl-1-piperazinyl)-benzaldehyde

This compound is prepared according to the methods of W. Nijhuis *et al*., Synthesis, 1987, 641-645 or that of J. Watthey *et al*., *J. Med*. *Chem*., **26**, pp. 1116-1122 (1983).

### Example 2

### 1-[4-(trifluoromethyl)phenyl]-2-pyrrolidinone

This compound is prepared by the method of W. C. Shakespeare, *Tetrahedron Letters,* **40**, 2035-2038 (1999), or that of T. Yamamoto and Y. Kurata, *Chemistry and Industry,* **1981**, pp. 737-738.

### Example 3

### 3-[[2-(4-methyl-1-piperazinyl)phenyl]methylene]-1-[4-(trifluoromethyl)phenyl]-2-pyrrolidinone

Under a nitrogen atmosphere, in a flame-dried round-bottomed flask equipped with a magnetic stirrer and reflux condenser, 2.8 g of sodium hydride (70 mmol of a 60% dispersion in mineral oil) was washed three times with hexanes to remove the oil. Anhydrous THF (50 mL) was added, stirring was begun and a mixture of 2-(4-methyl-1-piperazinyl)-benzaldehyde (9.6 g, 47 mmol) and 1-[4-(trifluoromethyl)phenyl]-2-pyrrolidinone (11.85 g, 51.7 mmol, Acros Organics, Pittsburgh, PA, catalog no. 27136-0010) in 200 mL THF was added dropwise over a 10 minute period. The mixture was then refluxed for 1.5 hours and allowed to cool to room temperature with stirring overnight. Water (25 mL) was added and the solvents were removed *in vacuo* to give a gummy solid which was partitioned between dichloromethane and saturated aqueous NH₄Cl and saturated NaHCO₃. The aqueous layer was further extracted with dichloromethane, the organic phases were combined and dried over Na₂SO₄, filtered and concentrated in vacuo to a solid. The solid was diluted in minimal dichloromethane and flash chromatographed on silica, eluting with ethyl acetate (100%), then 98:1:1 ethyl acetate: methanol: diethylamine. Fractions containing the product, as determined by thin layer chromatography, were combined and concentrated to give 16.1 g of pale yellow solids. Recrystallization yields pure title product. M.p. 186-189 °C. Mass spectrum: 416 (M⁺¹). ¹H-nmr (CDCl₃, 400 MHz) δ 7.92 (2H, d), 7.84 (1H, m), 7.64 (2H, d), 7.46 (1H, d), 7.33 (1H, m), 7.07 (2H, m), 3.96 (2H, t), 3.20 (2H, m), 2.99 (4H, m), 2.62 (4H, bs), 2.34 (3H, s). Elemental Analysis: Calcd. for C₂₃H₂₄F₃N₃O: C 66.49, H 5.82, N 10.11; Found: C 66.50, H 5.93, N 10.20.

### Example 4

### 3-[[2-(4-methyl-1-piperazinyl)phenyl]methyl]-1-[4-(trifluoromethyl)phenyl]-2-pyrrolidinone

**Method A:** A mixture of 3-[[2-(4-methyl-1-piperazinyl)phenyl]methylene]-1-[4-(trifluoromethyl)phenyl]-2-pyrrolidinone (200 mg, 0.482 mmol), ammonium formate (600 mg, 9.6 mmol) and 10% palladium on carbon (60 mg) in 17 mL of anhydrous methanol and 10 mL ethyl acetate was refluxed under N2 for 16 hours. After cooling, the catalyst was removed by filtration through diatomaceous earth (d.e.), the solvent was removed in vacuo and the residue was treated with saturated aqueous sodium bicarbonate and dichloromethane. The organic layer was removed, combined with a second extraction of the aqueous layer with additional dichloromethane, washed with saturated aqueous NaCI and dried. The solvent was again removed in vacuo to give the crude product as a white solid (135 mg). This solid was dissolved in hot ethyl acetate and crystallized by the addition of a few drops of hexanes. The title product, 71 mg, has a melting point of 110-111 °C. Mass spectrum: 418 (M⁺¹). The above free base in ethyl acetate, treated with 1.0 M HCl in diethyl ether, then hexanes produced white crystals of the hydrochloride salt, 67 mg. M.p. 181-183 °C. ¹H-nmr (DMSO-d₆, 400 MHz, HCl salt) δ 10.61 (1H, br s), 7.91 (2H, d), 7.72 (2H, d), 7.30-7.18 (2H, m), 7.18-7.03 (2H, m), 3.73 (2H, t), 3.50-3.33 (2H, m), 3.22-2.94 (8H, m), 2.78 (3H, s), 2.70 (1H, dd), 2.03 (1H, m), 1.74(1H, m). Elemental Analysis: Calcd. for C₂₃H₂₆F₃NO·HCl·0.5H₂O: C 59.67, H 6.10, N 9.08; Found: C 59.84, H 6.06, N 8.96.

**Method B:** A solution of 3-[[2-(4-methyl-1-piperazinyl)phenyl]methylene]-1-[4-(trifluoromethyl)phenyl]-2-pyrrolidinone (400 mg, 0.96 mmol) in 25 mL of methanol and 25 mL of tetrahydrofuran was combined with 170 mg of 10% palladium on carbon and hydrogenated on a Parr Shaker apparatus (Parr Instrument Co., Moline, IL) at 50 psi for a total of 24 hours. The catalyst was then removed by filtration though d.e. and the solvent was removed in vacuo to give a yellow gummy residue, 473 mg. Chromatography (silica gel) eluting with 5% methanol:95% ethyl acetate gave clean product, 310 mg, as yellow crystals. Thin-layer chromatography (tlc) on silica gel plates, eluting with 85:10:5 ethyl acetate: methanol: triethylamine showed this material identical in R_{f} to that produced by Method A. Mass spectrum: 418 (M⁺¹).

**Method C:** To a slurry of 300 mg (0.723 mmol) of 3-[[2-(4-methyl-1-piperazinyl)phenyl]methylene]-1-[4-(tri-fluoromethyl)phenyl]-2-pyrrolidinone in 25 mL of anhydrous methanol, under N₂ at room temperature, was added 165 mL (1.8 mmol) of samarium (II) iodide, (0.1 M SmI₂ in THF, Aldrich Chemical Co., Milwaukee, WI). After 0.5 hr another 15 mL of SmI₂ in THF was added and after another 0.5 hr an additional 5 mL of SmI₂ in THF was added. After stirring the mixture at room temperature overnight, a mass spectrum of an aliquot showed complete conversion to the reduced, title product. The solvent was removed in vacuo, the residue was partitioned between water and dichloromethane and filtered through diatomaceous earth (d.e.). The organics were dried over Na₂SO₄, filtered, concentrated in vacuo. The residue was flash chromatographed on silica gel eluting with 98:1:1 dichloromethane: methanol: diethylamine to give a tan solid, 1.78 g. M.p. 94-105 °C. Mass Spectrum: 418 (M⁺¹). ¹H-nmr of the free base was consistent with the compound obtained using method A.

### Example 5

### Chromatographic separation of (+)- and -(-)-3-[[2-(4-methyl-1-piperazinyl)phenyl]methyl]-1-[4-(trifluoromethyl)phenyl]-2-pyrrolidinone.

The crude racemic mixture (6.67g) prepared according to the preceding example (Method B) was separated using the following high pressure liquid chromatography (HPLC) procedure. The solid was dissolved in 12 mL of 1:1 methanol: dichloromethane and injected in three equal portions onto a Chiracel OJ column (10cm X 50 cm, µ packing, from Chiral Technologies Inc., Exton, PA). The column, attached to a Waters Prep LC2000 with System Controller and Waters 486 Tunable Absorbance Detector (Waters Corp., Milford, MA) set for UV max 250 nm was eluted with a mobile phase of 85:15:0.025 hexanes: isopropanol: diethylamine at a flow rate of 250 mL/min. Enantiomer 1, eluting at ∼ 30 min was concentrated to 3.05 g of white solids. Enantiomer 2, eluting at ∼ 45 min, was concentrated to 2.98 g of white solids.

Enantiomer 1 was dissolved in 50 mL of ethyl acetate and treated with 7.2 mL of 1N HCl in diethyl ether (Aldrich Chemical Co.) and stirred slowly at room temperature overnight to produce 2.37 g of white solids. M.p. 160-165 °C, [α]²⁰_{D} = +43.6° (c=13, methanol). Analysis calculated for C₂₃H₂₆F₃N₃O·HCl·H₂O: C, 58.53, H, 6.19, N, 8.90. Found: C, 58.20, H, 6.21, N, 8.87.

Enantiomer 2 was dissolved in 50 mL of ethyl acetate and treated with 7.0 mL of 1N HCl in diethyl ether (Aldrich Chemical Co.) and stirred slowly at room temperature overnight to produce 2.43 g of white solids. M.p. 165-169 °C, [α]²⁰_{D} = -43.9° (c=11, methanol). Analysis calculated for C₂₃H₂₆F₃N₃O·HCl·1.5H₂O: C, 57.44, H, 6.29, N, 8.74. Found: C, 57.57, H, 6.31, N, 8.76.

### Example 6

### 3(S)-[[2-(4-methyl-1-piperazinyl)phenyl]methyl]-1-[4-(trifluoromethyl)phenyl]-2-pyrrolidinone

To a solution of 3-[[2-(4-methyl-1-piperazinyl)phenyl]methyl]-1-[4-(trifluoromethyl)phenyl]-2-pyrrolidinone (3.82 g, 9.20 mmol) in 2-butanone (40 mL) at 50 °C was added di-*p*-toluoyl-D-tartaric acid (3.55 g, 9.20 mmol). The solution cooled to room temperature and was stirred overnight. The white solids were filtered to give 3(*S*)-[[2-(4-methyl-1-piperazinyl)phenyl]methyl]-1-[4-(trifluoromethyl)-phenyl]-2-pyrrolidinone di-*p*-toluoyl-D-tartrate (3.31 g, 45% yield, 90% of theory) as a 92:8 ratio of enantiomers by chiral HPLC. M.p. = 142-144 °C. [α]_{D} = +43.2° (c = 1.0 in MeOH). IR: 1722, 1612, 1323, 1122, 752 cm⁻¹. ¹H-nmr (400 MHz, CD₃OD): δ 1.82-1.92 (m, 1), 2.11-2.18 (m, 1), 2.39 (s, 6), 2.70 (dd, 1, J = 13.7, 10.0), 2.85 (s, 3), 2.99-3.14 (m, 6). 3.37 (dd, 4, J = 13.7, 4.1), 3.73-3.83 (m, 2), 5.89 (s, 2), 7.13 (t, 2, J = 7.9), 7.20-7.30 (m, 6), 7.68 (d, 2, J = 8.7), 7.88 (d, 2, J = 8.7), 8.00 (d, 4, J = 8.3). ¹³C-nmr (125 MHz, CD₃OD): δ 21.82, 25.67, 33.75, 43.69, 46.15, 48.14, 51.27, 55.31, 121.02, 122.40, 126.78, 126.89, 127.10, 127.36, 128.49, 129.12, 130.33, 131.22,132.05, 136.52, 144.24, 145.65, 151.45, 167.51, 178.61. Analysis: calculated for C₄₃H₄₄F₃N₃O₉: C, 64.25; H, 5.52; N, 5.23. Found: C, 63.85; H, 5.43; N, 5.26.

The above salt was dissolved in CH₂Cl₂ (20 mL) and the solution was washed with 1N aqueous sodium hydroxide (2 times with 15 mL), water (10 mL) and brine (10 mL). The organic layer was concentrated to afford the title compound as a solid.

### Example 7

### Racemization of 3(R)-[[2-(4-methyl-1-piperazinyl)phenyl]methyl]-1-[4-(trifluoromethyl)phenyl]-2-pyrrolidinone

A dichloromethane solution enriched in 3(*R*)-[[2-(4-methyl-1-piperazinyl)phenyl]methyl]-1-[4-(trifluoromethyl)phenyl]-2-pyrrolidinone di-*p*-toluoyl-D-tartrate (2.40g in 15 mL, 3.00 mmol) was washed with 1N aqueous sodium hydroxide (2 times with 10 mL), water (10 mL) and brine (10 mL). The organic layer was concentrated to afford the compound of formula XIV as a solid (1.25 g).

The crude solid described above was dissolved in THF (20 mL) and potassium t-butoxide (71 mg, 0.63 mmol) was added. The solution was heated to 65 °C for 12 hours. The reaction mixture was cooled and partitioned between water (10 mL) and CH₂Cl₂ (15 mL). The organic layer was washed with saturated NaHCO₃ (10 mL) and brine (10 mL), and concentrated to give the compound of formula VIII, a racemic mixture, as determined by chiral HPLC.

### Example 8

### Preparation of 3-(S)-(-)-[[2-(1-piperazinyl)phenyl]methyl]-1-(trifluoromethyl)phenyl]-2-pyrrolidinone

A mixture of 3-(*S*)-(-)-[[2-(4-methyl-1-piperazinyl)phenyl]methyl]-1-(trifluoromethyl)-phenyl]-2-pyrrolidinone (0.22 g, 0.53 mmol, enantiomer 2 as prepared in Example 5) and 1-chloroethyl chloroformate (0.068 mL, 0.63 mmol) in 6 mL of 1,2-dichloroethane was heated at reflux temperature for a total of 32 hours. A tlc (CHCl₃:CH₃OH:TEA, 90:10:2) at this time indicated a single new spot less polar than the starting material. The solvent was removed *in vacuo* and the residue was dissolved in 10 mL of methanol and refluxed for 1 hour. The solvent was removed *in vacuo* and the residue was subjected to flash chromatography on silica gel using ethyl acetate:methanol:TEA, 96:2:2 to elute the product fractions. Removal of the solvent left 73 mg of colorless oil, which was redissolved in 3 mL of ethyl acetate and treated with 0.2 mL of 1 N HCl in diethyl ether. After stirring at room temperature, the precipitated solids were filtered and dried under vacuum to give a white solid, 47 mg. M.p. 269-271 °C Analysis calculated for C₂₂H₂₄F₃N₃O·HCl·0.75H₂O: C, 58.28, H, 5.89, N, 9.27. Found: C, 58.28, H, 5.88, N, 9.23.

### Example 9

### 3-(S)-(-)-[[2-(4-(³H₃-Methyl)-1-piperazinyl)phenyl]methyl]-1-(trifluoromethyl)phenyl]-2-pyrrolidinone

3-(*S*)-(-)-[[2-(1-piperazinyl)phenyl]methyl]-1-(trifluoromethyl)phenyl]-2-pyrrolidinone (9 mg, 0.02 mmol) was dissolved in 0.3 mL of anhydrous N,N-dimethylformamide containing 40 mg of anhydrous potassium carbonate. Carrier-free ³H-iodomethane (C³H₃I, 0.02 mmol) was added and the reaction stirred at room temperature for 1 hr. The labile tritium was removed by evaporation of three portions of ethanol, leaving a crude material containing 1660 mCi. Reverse-phase tlc of this material (CH₃CN:1%TEAA, 1:1, pH 4) showed only a single radioactive component which co-migrated with non-tritiated product. The material was fractionated using a Zorbax Rx-C18 column, eluting with CH₃CN:1%TEAA, 1:1, pH 4 at a flow rate of 1 mL/min, with UV detection at 280 nm. The compound was converted to the HCl salt by addition of HCl (1 equivalent) to the pure fractions. ³H-nmr (CD₃OD): 85% at 3.20 ppm, 15% at 2.75 ppm. MS: (FAB): 83.0 Ci/mmol.

## Claims

1. A compound of formula II: 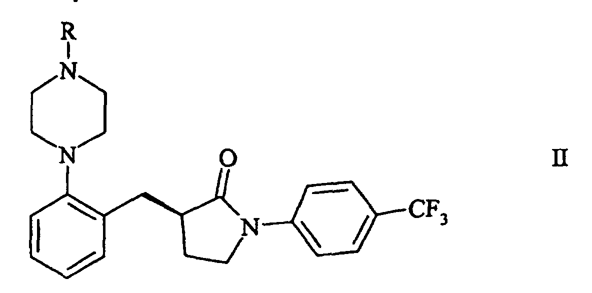 and pharmaceutically acceptable salts thereof; wherein the absolute stereochemistry at the 3-position is *(S)* and R is CH₃.

2. An enantiomeric mixture of the compound according to claim 1 and (+)-3(*R*)-[[2-(4-methyl-1-piperazinyl)phenyl]methyl]-1-[4-(trifluoromethyl)phenyl]-2-pyrrolidinone, or pharmaceutically acceptable salts thereof; wherein the ratio of the compound according to claim 1 to the *(R)*-enantiomer is in excess of 2:1.

3. A mixture according to claim 2 wherein the ratio is in excess of 5:1.

4. A mixture according to claim 2 wherein the ratio is 99:1 or greater.

5. A process for the preparation of the compound according to claim 1, comprising the steps of:
(i) dissolving a compound of the formula I: 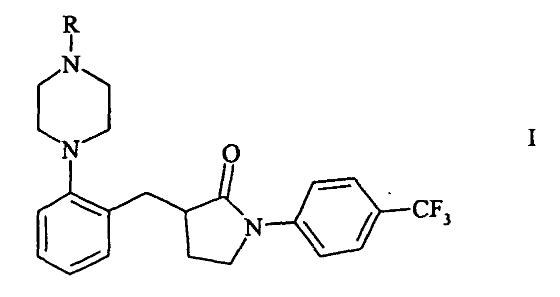 wherein R is CH₃, in an appropriate solvent in the presence of (+)-di-p-toluoyl-D-tartaric acid;
(ii) collecting the solid precipitate comprising the salt of formula III: 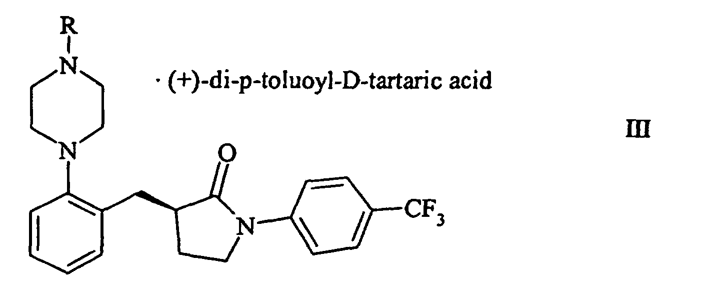 wherein R is CH₃, and
(iii) treating said precipitate with a base.

6. A process according to claim 5 wherein the appropriate solvent in step (i) is selected from the group consisting of 2-butanone, acetone, 3-pentanone, methanol, ethanol, isopropanol, and ethyl acetate.

7. A process according to claim 5 wherein the base is selected from the group consisting of sodium hydroxide, sodium carbonate, sodium bicarbonate, potassium hydroxide, potassium carbonate, potassium bicarbonate, lithium hydroxide, and ammonium hydroxide.

8. A pharmaceutical composition comprising a compound according to claim 1 and a pharmaceutically acceptable carrier.

9. Use of a compound according to claim 1 in the manufacture of a medicament for treating a disease, disorder or condition selected from depression, generalized anxiety disorder, phobias, post-traumatic stress syndrome, avoidant personality disorder, premature ejaculation, eating disorders, obesity, chemical dependencies, Alzheimer's disease, obsessive-compulsive disorder, panic disorder, memory disorders, Parkinson's diseases, endocrine disorders, and gastrointestinal tract disorders where changes in motility and secretion are involved, in a mammal.

10. Use of a compound according to claim 1 in the manufacture of a medicament for treating a disease, disorder or condition that can be treated by enhancing serotonergic neurotransmission in a mammal.

11. Use according to claim 9 or claim 10 wherein the amount of a compound according to claim 1 is a serotonin receptor antagonizing effective amount.

12. A pharmaceutical composition for treating a disease, disorder or condition that can be treated by enhancing serotonergic neurotransmission in a mammal, comprising:
a) a pharmaceutically acceptable carrier;
b) a compound according to claim 1; and
c) a 5-HT re-uptake inhibitor or a pharmaceutically acceptable salt thereof;
wherein the amount of the active compounds are such that the combination is effective in treating such disease, disorder or condition.

13. Use of a compound according to claim 1 and a 5-HT re-uptake inhibitor or a pharmaceutically acceptable salt thereof in the manufacture of a medicament for treating a disease, disorder or condition that can be treated by enhancing serotonergic neurotransmission in a mammal, wherein the amounts of the active compounds are such that the combination is effective in treating such disease, disorder or condition.

14. Use of a 5-HT_{1A} antagonist or a pharmaceutically acceptable salt thereof and a compound according to claim 1 or a pharmaceutically acceptable salt thereof; in the manufacture of a medicament for treating a disease, disorder or condition that can be treated by enhancing serotonergic neurotransmission in a mammal, wherein the amounts of the active compounds are such that the combination is effective in treating such disease, disorder or condition.

15. A pharmaceutical composition according to claim 12, wherein the 5-HT re-uptake inhibitor is sertraline or a pharmaceutically acceptable salt thereof.

16. A use according to claim 13, wherein the 5-HT re-uptake inhibitor is sertraline or a pharmaceutically acceptable salt thereof.

17. Use of a compound according to claim 1 anda 5-HT re-uptake inhibitor or a pharmaceutically acceptable salt thereof, in the manufacture of a medicamentfor treating a disease, disorder or condition selected from depression, generalized anxiety disorder, phobias, post-traumatic stress syndrome, avoidant personality disorder, sexual dysfunction, eating disorders, obesity, chemical dependencies, Alzheimer's disease, obsessive-compulsive disorder, panic disorder, memory disorders, Parkinson's diseases, endocrine disorders, and gastrointestinal tract disorders where changes in motility and secretion are involved, in a mammal, wherein the amounts of the active compounds are such that the combination is effective in treating such disorder or condition.

18. Use of a 5-HT_{1A} antagonist or a pharmaceutically acceptable salt thereof and a compound according to claim 1 or a pharmaceutically acceptable salt thereof, in the manufacture of a medicament for treating a disorder or condition selected from depression, generalized anxiety disorder, phobias, post-traumatic stress syndrome, avoidant personality disorder, sexual dysfunction, eating disorders, obesity, chemical dependencies, Alzheimer's disease, obsessive-compulsive disorder, panic disorder, memory disorders, Parkinson's diseases, endocrine disorders, and gastrointestinal tract disorders where changes in motility and secretion are involved, in a mammal, wherein the amounts of the active compounds are such that the combination is effective in treating such disease, disorder or condition.

19. A compound according to claim 1 for use as a medicament.

20. A compound according to claim 1 having an IC₅₀ less than 10.0 nM for 5-HT_{1D}.

21. A compound according to claim 1 having an IC₅₀ less than 400 nM for 5-HT_{1A}.

22. A process according to claim 5, wherein in the compound of formula I any of the carbon atoms is ¹¹C, any of the hydrogen atoms is ³H or any of the fluorine atoms is ¹⁸F.

23. A compound of formula V: 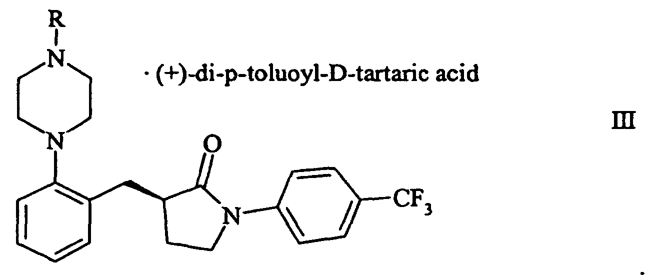 wherein R is CH₃.

24. A compound according to claim 1 wherein one or more hydrogen atoms is ³H.

25. . A compound according to claim 1 wherein R is -C³H₃.

26. A compound according to claim 1 wherein one or more of the fluorine atoms is ¹⁸F.

27. A compound according to claim 1 wherein one or more of the carbon atoms is ¹¹C or ¹⁴C.

28. A compound according to claim 1 one or more of the hydrogen atoms is ³H, one or more of the carbon atoms is ¹¹C or ¹⁴C, and one or more of the fluorine atoms is ¹⁸F; or any combination thereof.

29. A method for the determination of the distribution in a tissue sample of a compound according to claim 1, comprising incubating a compound according to claim 28 with said tissue sample.

## Patentansprüche

1. Eine Verbindung der Formel II: 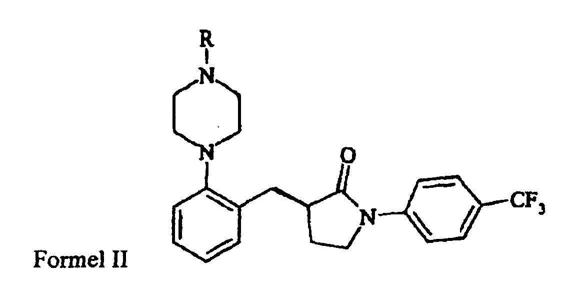 und deren pharmazeutisch akzeptable Salze, wobei *(S)* die absolute sterische Konfiguration in der 3-Stellung ist und R eine CH₃-Gruppe darstellt.

2. Eine enantiomere Mischung der Verbindung gemäß Anspruch 1 und (+)-3(R)-[[2-(4-methyl-1-piperazinyl)phenyl]methyl]-1-[4-(trifluoromethyl)phenyl]-2-pyrrolidon oder pharmazeutischer akzeptabler Salze hiervon, wobei das Verhältnis der Verbindung gemäß Anspruch 1 zu dem *(R)*-Enantiomeren im Überschuss von 2:1 vorliegt.

3. Eine Mischung entsprechend Anspruch 2 wobei das Verhältnis im Überschuss von 5:1 vorliegt.

4. Eine Mischung entsprechend Anspruch 2 wobei das Verhältnis 99:1 oder größer ist.

5. Ein folgende Schritte umfassendes Verfahren zur Herstellung der Verbindung gemäß Anspruch 1:
(i) Lösen einer Verbindung der Formel I: 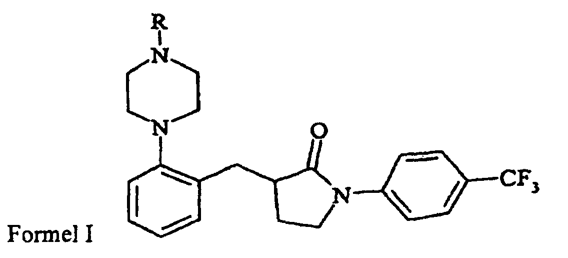 wobei R eine CH₃-Gruppe ist in einem geeigneten Lösungsmittel in Gegenwart von (+)-di-p-toluoyl-D-Weinsäure;
(ii) aufsammeln des festen Niederschlages, der das Salz gemäß Formel III umfaßt: 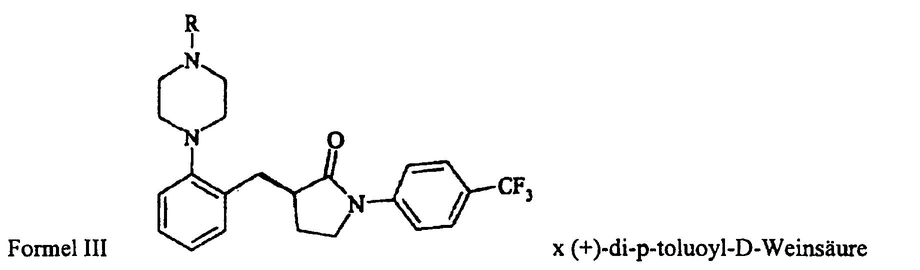 wobei R eine CH₃-Gruppe ist und
(iii) behandeln des besagten Niederschlages mit einer Base.

6. Ein Verfahren gemäß Anspruch 5 wobei das geeignete Lösungsmittel in Stufe (i) aus der aus 2-Butanon, Aceton, 3-Pentanon, Methanol, Ethanol, Isopropanol und Ethylacetat bestehenden Gruppe ausgewählt ist.

7. Ein Verfahren gemäß Anspruch 5 wobei die Base aus der aus Natriumhydroxid, Natriumcarbonat, Natriumbicarbonat, Kaliumhydroxid, Kaliumcarbonat, Kaliumbicarbonat, Lithiumhydroxid und Ammoniumhydroxid bestehenden Gruppe ausgewählt ist.

8. Eine pharmazeutische Zubereitung umfassend eine Verbindung entsprechend Anspruch 1 und einen pharmazeutisch sinnvollen Träger.

9. Die Verwendung einer Verbindung entsprechend Anspruch 1 bei der Herstellung eines Arzneimittels zur Behandlung einer Erkrankung, Funktionsstörung oder Zustandes beim Säuger, ausgewählt aus Depression, generelle Angststörungen, Phobien, posttraumatisches Stress-Syndrom, psychotische Persönlichkeitsstörung, vorzeitige Ejakulation, Essstörungen, Fettsucht, hemikalienabhängigkeiten, Alzheimererkrankung, Zwangsneurose, Panikattacken, Gedächtnisstörungen, Parkinsonerkrankungen, endokrine Störungen und Störungen des Magen-Darm-Traktes, wobei Veränderungen in der Beweglichkeit und der Sekretion beteiligt sind.

10. Die Verwendung einer Verbindung entsprechend Anspruch 1 zur Herstellung eines Arzneimittels zur Behandlung einer Erkrankung, Funktionsstörung oder Zustandes eines Säugers, um die serotoninogene Erregungsübertragung zwischen den Nervenzellen zu verbessern.

11. Die Anwendung gemäß Anspruch 9 oder Anspruch 10 wobei die Menge der Verbindung gemäß Anspruch 1 eine zum Serotoninrezeptor antagonistisch wirksame Menge ist.

12. Eine pharmazeutische Zubereitung zur Behandlung einer Erkrankung, einer Funktionsstörung oder eines Zustandes zur Verbesserung der serotoninogenen Erregungsübertragung zwischen den Nervenzellen bei Säugern, umfassend:
a) einen pharmazeutisch akzeptablen Träger
b) eine Verbindung gemäß Anspruch 1; und
c) einen 5-HT Wiederaufnahmehemmer oder ein pharmazeutisch akzeptables Salz hiervon;
wobei die Menge der wirksamen Bestandteile dergestalt ist, dass bei der Behandlung einer derartigen Erkrankung, Funktionsstörung oder Zustandes die Kombination wirksam ist.

13. Die Verwendung einer Verbindung gemäß Anspruch 1 und eines 5-HT-Wiederaufnahmehemmers oder eines pharmazeutisch akzeptablen Salzes hiervon bei der Herstellung eines Arzneimittels zur Behandlung einer Erkrankung, Funktionsstörung oder Zustandes bei Säugern in der Weise, indem die serotoninogene Erregungsübertragung zwischen den Nervenzellen verbessert wird, wobei die Menge der wirksamen Bestandteile dergestalt sind, dass deren Kombination bei der Behandlung einer solchen Erkrankung, Funktionsstörung oder Zustand wirksam ist.

14. Die Verwendung eines 5-HT_{1A}-Antagonisten oder ein pharmazeutisch akzeptables Salz hiervon und eine Verbindung gemäß Anspruch 1 oder ein pharmazeutisch akzeptables Salz hiervon bei der Herstellung eines Arzneimittels zur Behandlung einer Erkrankung, Funktionsstörung oder Zustandes bei Säugern in der Weise, indem die serotoninogene Erregungsübertragung zwischen den Nervenzellen verbessert wird, wobei die Menge der wirksamen Bestandteile dergestalt sind, dass deren Kombination bei der Behandlung einer solchen Erkrankung, Funktionsstörung oder Zustandes wirksam ist.

15. Eine pharmazeutische Zubereitung gemäß Anspruch 12, worin der 5-HT-Wiederaufnahmehemmer Sertralin oder ein pharmazeutisch akzeptables Salz hiervon ist.

16. Eine pharmazeutische Zubereitung gemäß Anspruch 13, worin der 5-HT-Wiederaufnahmehemmer Sertralin oder ein pharmazeutisch akzeptables Salz hiervon ist.

17. Die Verwendung einer Verbindung gemäß Anspruch 1 und ein 5-HT-Wiederaufnahmehemmer oder ein pharmazeutisch akzeptables Salz hiervon bei der Herstellung eines Arzneimittels zur Behandlung einer Erkrankung, Funktionsstörung oder Zustandes beim Säuger, ausgewählt aus Depression, generellen Angststörungen, Phobien, posttraumatischem Stress-Syndrom, psychopatischer Persönlichkeitsstörung, sexueller Funktionsstörung, Essstörungen, Fettsucht, Chemikalienabhängigkeiten, Alzheimererkrankung, Zwangsneurose, Panikattacken, Gedächtnisstörungen, Parkinsonerkrankungen, endokrine Störungen und Störungen des Magen-Darm-Traktes, in Verbindung mit Veränderungen in der Beweglichkeit und der Sekretion, wobei die Menge der wirksamen Bestandteile dergestalt sind, dass deren Kombination bei der Behandlung solcherlei Erkrankung, Funktionsstörung oder Zustandes wirksam ist.

18. Die Verwendung eines 5-HT_{1A} -Antagonisten oder eines pharmazeutisch akzeptablen Salzes hiervon und einer Verbindung gemäß Anspruch 1 oder ein pharmazeutisch akzeptablen Salz hiervon bei der Herstellung eines Arzneimittels zur Behandlung einer Erkrankung oder Zustandes beim Säuger, ausgewählt aus Depression, generellen Angststörungen, Phobien, posttraumatischem Stress-Syndrom, psychopatischer Persönlichkeitsstörung, sexueller Funktionsstörung, Essstörungen, Fettsucht, Chemikalienabhängigkeiten, Alzheimererkrankung, Zwangsneurose, Panikattacken, Gedächtnisstörungen, Parkinsonerkrankungen, endokrine Störungen und Störungen des Magen-Darm-Traktes, in Verbindung mit Veränderungen in der Beweglichkeit und der Sekretion, wobei die Menge der wirksamen Bestandteile dergestalt sind, dass deren Kombination bei der Behandlung solcherlei Erkrankung, Funktionsstörung oder Zustandes wirksam ist.

19. Eine Verbindung gemäß Anspruch 1 zur Verwendung als Arzneimittel.

20. Eine Verbindung gemäß Anspruch 1, die einen IC₅₀ für 5-HT_{1D} von weniger als 10,0 nM aufweist.

21. Eine Verbindung gemäß Anspruch 1, die einen IC₅₀ für 5-HT_{1A} von weniger als 400 nM aufweist.

22. Ein Verfahren gemäß Anspruch 5 worin in Formel I jedes der Kohlenstoffatome aus ¹¹C, jedes der Wasserstoffatome aus ³H oder jedes Fluoratom aus ¹⁸F besteht.

23. Eine Verbindung der Formel V 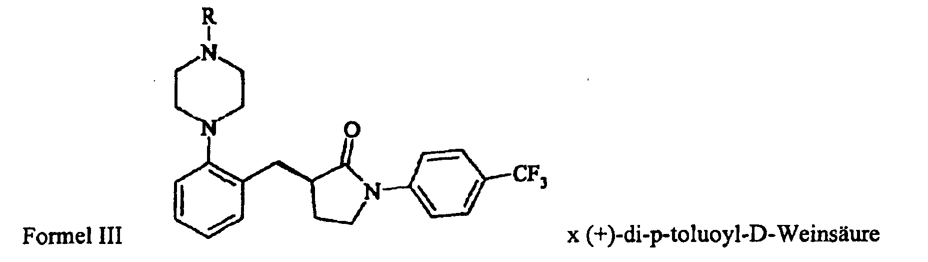 wobei R eine CH₃-Gruppe ist.

24. Eine Verbindung gemäß Anspruch 1, wobei ein oder mehrere Wasserstoffatome ³H sein können.

25. Eine Verbindung gemäß Anspruch 1, wobei R eine -C³H₃ Gruppe ist.

26. Eine Verbindung gemäß Anspruch 1, wobei ein oder mehrere Fluoratome ¹⁸F sein können.

27. Eine Verbindung gemäß Anspruch 1, wobei ein oder mehrere Kohlenstoffatome ¹¹C oder ¹²C sein können.

28. Eine Verbindung gemäß Anspruch 1, wobei ein oder mehrere Wasserstoffatome ³H sind, ein oder mehrere Kohlenstoffatome ¹¹C oder ¹⁴C und ein oder mehrere Fluoratome ¹⁸F oder jegliche Kombination hiervon sein können.

29. Eine Methode zur Bestimmung der Verteilung einer Verbindung gemäß Anspruch 1 in einer Gewebeprobe, umfassend das Inkubieren einer Verbindung gemäß Anspruch 28 mit besagter Gewebeprobe.

## Revendications

1. Composé de formule II : 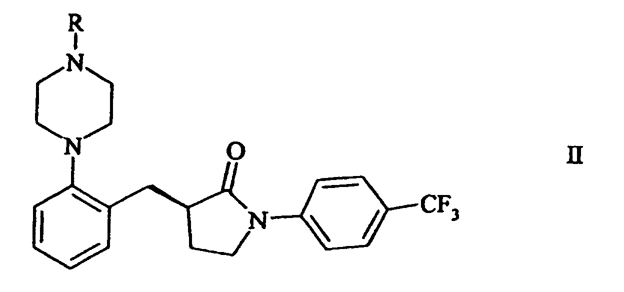 et ses sels pharmaceutiquement acceptables ; dans lesquels la stéréochimie absolue en position 3 est la stéréochimie *(S)* et R représente un groupe CH₃.

2. Mélange d'énantiomères du composé suivant la revendication 1 et de (+)-3*(R)*-[[2-(4-méthyl-1-pipérazinyl)-phényl]méthyl]-1-[4-(trifluorométhyl)phényl]-2-pyrrolidinone, ou de ses sels pharmaceutiquement acceptables ; dans lequel le rapport du composé suivant la revendication 1 à l'énantiomère (R) est supérieur à 2:1.

3. Mélange suivant la revendication 2, dans lequel le rapport est supérieur à 5:1.

4. Mélange suivant la revendication 2, dans lequel le rapport est égal ou supérieur à 99:1.

5. Procédé pour la préparation du composé suivant la revendication 1, comprenant les étapes consistant :
(i) à dissoudre un composé de formule I : 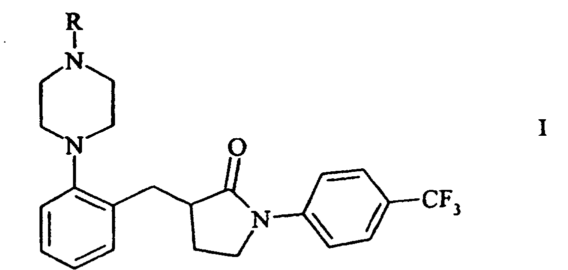 dans laquelle R représente un groupe CH₃, dans un solvant approprié en présence d'acide (+)-di-p-toluoyl-D-tartrique ;
(ii) à recueillir le précipité solide comprenant le sel de formule III : 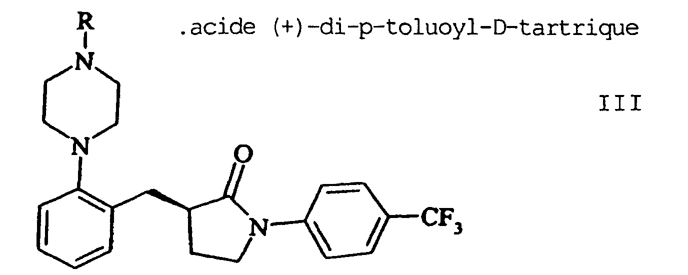 dans laquelle R représente un groupe CH₃, et
(iii) à traiter ledit précipité avec une base.

6. Procédé suivant la revendication 5, dans lequel le solvant approprié dans l'étape (i) est choisi dans le groupe consistant en 2-butanone, acétone, 3-pentanone, méthanol, éthanol, isopropanol et acétate d'éthyle.

7. Procédé suivant la revendication 5, dans lequel la base est choisie dans le groupe consistant en hydroxyde de sodium, carbonate de sodium, bicarbonate de sodium, hydroxyde de potassium, carbonate de potassium, bicarbonate de potassium, hydroxyde de lithium et hydroxyde d'ammonium.

8. Composition pharmaceutique comprenant un composé suivant la revendication 1 et un support pharmaceutiquement acceptable.

9. Utilisation d'un composé suivant la revendication 1 dans la production d'un médicament destiné au traitement d'une maladie, d'un trouble ou d'une affection choisi entre la dépression, le syndrome d'anxiété généralisée, les phobies, le syndrome de stress post-traumatique, le syndrome d'évitement affectif, l'éjaculation précoce, des troubles de l'alimentation, l'obésité, des dépendances chimiques, la maladie d'Alzheimer, le syndrome obsessionnel compulsif, les attaques de panique, des troubles de la mémoire, la maladie de Parkinson, des troubles endocriniens et des troubles du tractus gastro-intestinal impliquant des modifications de la motilité et de la sécrétion, chez un mammifère.

10. Utilisation d'un composé suivant la revendication 1 dans la production d'un médicament destiné au traitement d'une maladie, d'un trouble ou d'une affection qui peut être traité en augmentant la neurotransmission sérotoninergique chez un mammifère.

11. Utilisation suivant la revendication 9 ou la revendication 10, dans laquelle la quantité d'un composé suivant la revendication 1 est une quantité efficace pour antagoniser le récepteur de sérotonine.

12. Composition pharmaceutique pour le traitement d'une maladie, d'un trouble ou d'une affection qui peut être traité en augmentant la neurotransmission sérotoninergique chez un mammifère, comprenant :
a) un support pharmaceutiquement acceptable ;
b) un composé suivant la revendication 1 ; et
c) un inhibiteur de réabsorption de 5-HT ou un de ses sels pharmaceutiquement acceptables ;
dans laquelle les quantités des composés actifs sont telles que l'association soit efficace dans le traitement de cette maladie, de ce trouble ou de cette affection.

13. Utilisation d'un composé suivant la revendication 1 et d'un inhibiteur de réabsorption de 5-HT ou d'un de ses sels pharmaceutiquement acceptables, dans la production d'un médicament destiné au traitement d'une maladie, d'un trouble ou d'une affection qui peut être traité en augmentant la neurotransmission sérotoninergique chez un mammifère, dans laquelle les quantités des composés actifs sont telles que l'association soit efficace dans le traitement de cette maladie, de ce trouble ou de cette affection.

14. Utilisation d'un antagoniste de 5-HT_{1A} ou d'un de ses sels pharmaceutiquement acceptables et d'un composé suivant la revendication 1 ou d'un de ses sels pharmaceutiquement acceptables, dans la production d'un médicament destiné au traitement d'une maladie, d'un trouble ou d'une affection qui peut être traité en augmentant la neurotransmission sérotoninergique chez un mammifère, dans laquelle les quantités des composés actifs sont telles que l'association soit efficace dans le traitement de cette maladie, de ce trouble ou de cette affection.

15. Composition pharmaceutique suivant la revendication 12, dans laquelle l'inhibiteur de réabsorption de 5-HT est la sertraline ou un de ses sels pharmaceutiquement acceptables.

16. Utilisation suivant la revendication 13, dans laquelle l'inhibiteur de réabsorption de 5-HT est la sertraline ou un de ses sels pharmaceutiquement acceptables.

17. Utilisation d'un composé suivant la revendication 1 et d'un inhibiteur de réabsorption de 5-HT ou d'un de ses sels pharmaceutiquement acceptables dans la production d'un médicament destiné au traitement d'une maladie, d'un trouble ou d'une affection choisi entre la dépression, le syndrome d'anxiété généralisée, les phobies, le syndrome de stress post-traumatique, le syndrome d'évitement affectif, un dysfonctionnement sexuel, des troubles de l'alimentation, l'obésité, des dépendances chimiques, la maladie d'Alzheimer, un syndrome obsessionnel compulsif, les attaques de panique, des troubles de la mémoire, la maladie de Parkinson, des troubles endocriniens et des troubles du tractus gastro-intestinal impliquant des modifications de la motilité et de la sécrétion, chez un mammifère, dans laquelle les quantités de composés actifs sont telles que l'association soit efficace dans le traitement de ce trouble ou de cette affection.

18. Utilisation d'un antagoniste de 5-HT_{1A} ou d'un de ses sels pharmaceutiquement acceptables et d'un composé suivant la revendication 1 ou d'un de ses sels pharmaceutiquement acceptables dans la production d'un médicament destiné au traitement d'un trouble ou d'une affection choisi entre la dépression, le syndrome d'anxiété généralisée, les phobies, le syndrome de stress post-traumatique, le syndrome d'évitement affectif, un dysfonctionnement sexuel, des troubles de l'alimentation, l'obésité, des dépendances chimiques, la maladie d'Alzheimer, le syndrome obsessionnel compulsif, les attaques de panique, des troubles de la mémoire, la maladie de Parkinson, des troubles endocriniens et des troubles du tractus gastro-intestinal impliquant des modifications de la motilité et de la sécrétion, chez un mammifère, dans laquelle les quantités des composés actifs sont telles que l'association soit efficace dans le traitement de cette maladie, trouble ou affection.

19. Composé suivant la revendication 1, destiné à être utilisé comme médicament.

20. Composé suivant la revendication 1, ayant une valeur de CI₅₀ inférieure à 10,0 nM pour 5-HT_{1D}.

21. Composé suivant la revendication 1, ayant une valeur de CI₅₀ inférieure à 400 nM pour 5-HT_{1A}.

22. Procédé suivant la revendication 5, dans lequel, dans le composé de formule I, n'importe lequel des atomes de carbone est un atome de ¹¹C, n'importe lequel des atomes d'hydrogène est un atome de ³H ou n'importe lequel des atomes de fluor est un atome de ¹⁸F.

23. Composé de formule V : 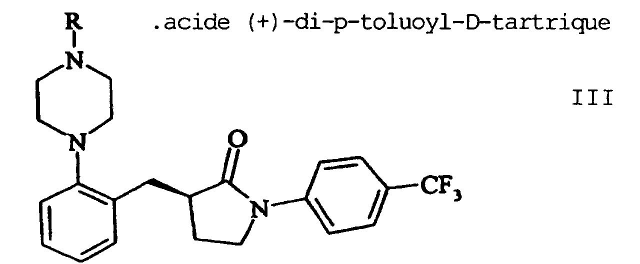 dans laquelle R représente CH₃.

24. Composé suivant la revendication 1, dans lequel un ou plusieurs atomes d'hydrogène sont des atomes de ³H.

25. Composé suivant la revendication 1, dans lequel R représente un groupe -C³H₃.

26. Composé suivant la revendication 1, dans lequel un ou plusieurs des atomes de fluor sont des atomes de ¹⁸F.

27. Composé suivant la revendication 1, dans lequel un ou plusieurs des atomes de carbone sont des atomes de ¹¹C ou ¹⁴C.

28. Composé suivant la revendication 1, dans lequel un ou plusieurs des atomes d'hydrogène sont des atomes de ³H, un ou plusieurs des atomes de carbone sont des atomes de ¹¹C ou ¹⁴C et un ou plusieurs des atomes de fluor sont des atomes de ¹⁸F ; ou comprenant n'importe laquelle de leurs associations.

29. Méthode pour déterminer la distribution dans un échantillon de tissu d'un composé suivant la revendication 1, comprenant la mise en incubation d'un composé suivant la revendication 28 avec ledit échantillon de tissu.
